# EUROPÄISCHE PATENTSCHRIFT

(11) **EP 3 216 408 B1**
(45) Veröffentlichungstag und Bekanntmachung des Hinweises auf die Patenterteilung: **08.05.2019**
(21) Anmeldenummer: 17159862.6
(22) Anmeldetag: 08.03.2017
(51) Int. Cl.: A61B 17/88, B01F 5/00, B05C 17/01, B01F 3/10

(54) **LAGERUNGS- UND MISCHSYSTEM FÜR PASTENFÖRMIGE ZEMENTKOMPONENTEN UND VERFAHREN DAFÜR**
STORAGE AND MIXING SYSTEM FOR PASTY CEMENT COMPONENTS AND METHOD THEREFOR
SYSTÈME DE STOCKAGE ET DE MÉLANGE POUR COMPOSANTS DE CIMENT PÂTEUX ET PROCÉDÉ ASSOCIÉ

(30) Priorität: 10.03.2016 DE 102016104409
(43) Veröffentlichungstag der Anmeldung: 13.09.2017
(73) Patentinhaber: Heraeus Medical GmbH, 61273 Wehrheim (DE)
(72) Erfinder: Vogt, Sebastian, 99092 Erfurt (DE); Kluge, Thomas, 56179 Vallendar (DE)
(74) Vertreter: Schultheiss & Sterzel Patentanwälte PartG mbB

(56) Entgegenhaltungen:
- EP-A2- 0 119 847
- EP-B1- 1 392 450
- WO-A1-2011/137971
- DE-A1- 2 521 392
- DE-U1-202014 102 416

## Beschreibung

Die Erfindung betrifft eine Lagerungs- und Mischsystem für pastenförmige Zweikomponenten-Polymethylmethacrylat-Knochenzemente, das Lagerungs- und Mischsystem aufweisend eine röhrenförmige Kartusche mit einem zylindrischen Innenraum.

Die Erfindung betrifft auch ein Verfahren zur Vermischung von pastenförmigen Zementkomponenten eines Zementteigs, insbesondere eines pastenförmigen Polymethylmethacrylat-Knochenzements, mit einem solchen Lagerungs- und Mischsystem.

Gegenstand der Erfindung ist somit ein einfaches, kostengünstig herzustellendes Lagerungs- und Mischsystem für pastenförmige Zweikomponenten-Polymethylmethacrylat-Knochenzemente, mit dem die hochviskosen, pastenförmigen Komponenten des Polymethylmethacrylat-Knochenzements auch mit manuell bedienbaren Austragsvorrichtungen gemischt und ausgetragen werden können. Mit dem Lagerungs- und Mischsystem sollen hochviskose, pastenförmige Komponenten von Zweikomponenten-Polymethylmethacrylat-Knochenzementen mit Hilfe von manuell bedienbaren Auspressvorrichtungen gemischt und ausgetragen werden können.

Konventionelle Polymethylmethacrylat-Knochenzemente (PMMA-Knochenzemente) sind aus einer pulverförmigen Komponente und einer flüssigen Monomerkomponente zusammengesetzt (K.-D. Kühn: Knochenzemente für die Endoprothetik: Ein aktueller Vergleich der physikalischen und chemischen Eigenschaften handelsüblicher PMMA-Zemente. Springer-Verlag Berlin Heidelberg New York, 2001). Diese Polymethylmethacrylat-Knochenzemente werden nach Vermischung des Zementpulvers mit der flüssigen Monomerkomponente im noch nicht ausgehärteten, pastenförmigen Zustand als Zementteig appliziert. Bei Verwendung von Mischsystemen befindet sich der Zementteig im Fall von Pulver-Flüssigkeitszementen in einer Kartusche. Aus dieser Kartusche wird der Zementteig durch Bewegung eines Austragskolbens heraus gedrückt. Die Austragskolben haben üblicherweise einen Durchmesser zwischen 30 mm bis 40 mm und damit an der Außenseite, an welcher der Stößel der Austragsvorrichtung beim Auspressvorgang angreift, eine Fläche von 7,0 cm² bis 12,5 cm². Die Bewegung des Austragskolbens wird durch manuell bedienbare, mechanische Austragsvorrichtungen bewirkt, die auch als Applikatoren bezeichnet werden. Diese Austragsvorrichtungen beziehungsweise Applikatoren haben normalerweise eine Auspresskraft im Bereich von ungefähr 1,5 kN bis 3,5 N.

Eine neuere Entwicklung stellen pastenförmige Zweikomponenten-Knochenzemente dar, wie sie beispielsweise aus der DE 10 2007 050 762 B3, der DE 10 2008 030 312 A1 und der DE 10 2007 052 116 B4 bekannt sind. Bei diesen Zweikomponenten-Knochenzementen werden zwei pastenförmige Zementkomponenten in zwei separaten Kartuschen mit zwei separaten Austragskolben aufbewahrt. Bei der Applikation werden beide Pasten durch Bewegung der Austragkolben aus den Kartuschen in einen statischen Mischer gepresst und nach erfolgter Vermischung durch ein Austragsrohr ausgetragen. Bei geeigneter Zusammensetzung der pastenförmigen Zementkomponenten wird nach der Vermischung der beiden Zementkomponenten sofort ein klebfreier, applikationsbereiter Zementteig erhalten. Wartezeiten bis zum Erreichen der Klebfreiheit des Zementteigs, die bei bisherigen konventionellen Polymethylmethacrylat-Knochenzementen immer zwangsläufig auftreten, entfallen dadurch. Wertvolle OP-Zeit kann dadurch gespart werden. WO2011/137971 offenbart ein Mischsystem für Knochenzemente, das aus zwei Kartuschen mit verbundenen Förderkolben und einem Austragsrohr mit einem Mischer besteht, wobei die Zementkomponenten aus den Kartuschen in das Rohr gedrückt und dort gemischt werden.

Eigene Versuche im Rahmen der vorliegenden Erfindung zeigten, dass während des Auspressvorgangs der Kartuschen auf Grund der hohen Viskosität der pastenförmigen Zementkomponenten ein sehr großer Druckabfall am statischen Mischer im Austragsrohr stattfindet. Eigene Versuche zeigten weiterhin, dass bei einem konischen Austragsrohr und einer Gesamtlänge von ca. 17 cm und mit einem Innendurchmesser von 11 mm am Kartuschenkopf und unter Verwendung von zehn statischen Mischelementen eine Auspresskraft größer 7 kN notwendig ist, um die hochviskosen Zementpasten in einer für den medizinischen Anwender akzeptablen Austragsgeschwindigkeit auszupressen.

Bei der Applikation der bisherigen, konventionellen PMMA-Knochenzemente, die aus einer flüssigen Monomerkomponente und einer separat dazu gelagerten Zementpulverkomponente bestehen, wird nach der Vermischung der beiden Zementkomponenten in Zementiersystemen beziehungsweise Vakuumzementiersystemen der gebildete Zementteig mit Hilfe von manuell bedienbaren Austragsvorrichtungen ausgepresst. Diese einfachen mechanischen Austragsvorrichtungen nutzen insbesondere Klemmstangen zum Auspressen, die durch einen manuell zu betätigenden Kipphebel angetrieben werden. Die manuell angetriebenen Austragsvorrichtungen sind seit Jahrzehnten weltweit erprobt und stellen bisher den Stand der Technik dar. Vorteilhaft an diesen Austragsvorrichtungen ist, dass der medizinische Anwender über die aufzubringende Handkraft ein Gefühl für den Eindringwiderstand des Knochenzementteigs in die Knochenstrukturen (Spongiosa) hat.

Bei hochviskosen pastenförmigen Zementkomponenten unter Verwendung von Kartuschen, bei denen die Austragskolben an den äußeren Kolbenseiten, an denen die Stößel der Austragsvorrichtungen angreifen, eine gesamte Fläche im Bereich von 7,0 cm² bis 12,5 cm² haben, sind diese Vorrichtungen nicht oder nur mit einem sehr großen Kraftaufwand manuell zu bedienen. Dieser große Kraftaufwand ist medizinischen Anwendern im OP nicht zuzumuten.

Aus dem Kleb- und Dichtstoffbereich sind auch elektrisch angetriebene Auspressvorrichtungen bekannt. Diese Vorrichtungen können sowohl mit Akkumulatoren und mit Batterien als auch mit Hilfe einer stationären Stromversorgung angetrieben werden. Diese Vorrichtungen können mit ihren zum Teil sehr großen Auspresskräften besonders zähe pastenförmige Massen auspressen. Nachteilig ist jedoch bei der Verwendung von Elektromotoren, dass diese Buntmetalle enthalten und kostenintensiv in der Beschaffung sind. Im steril zu haltenden OP-Bereich müssen derartige Vorrichtungen aufwendig sterilisiert oder sogar ausgetauscht werden. Bei einer elektrischen Verkabelung kann die Bewegung des Anwenders im OP behindert werden.

Weiterhin wurden auch pneumatische Vorrichtungen vorgeschlagen. Diese Geräte erfordern einen stationären oder mobilen Druckluftanschluss (US 2 446 501 A; DE 20 2005 010 206 U1). Dazu sind Druckluftschläuche notwendig, welche die Bewegung des Anwenders behindern können.

Alternativ dazu ist auch die Verwendung von Druckgaspatronen zur Bereitstellung von Druckgas möglich. Dazu wurden Vorrichtungen vorgeschlagen, bei denen der Druckgaszufluss durch ein Ventil und zusätzlich durch ein zweites Ventil der Strom der viskosen Masse gesteuert werden (US 2004/0074927 A1; US 6 935 541 B1). Bei diesen Vorrichtungen sind die Gaspatronen in den Vorrichtungen integriert. Bei derartigen an Druckluft angeschlossenen oder Druckgaspatronen enthaltenden Systemen ist immer eine Druckgasquelle erforderlich, ohne die die Systeme nicht mehr anwendbar sind.

Die US 8,544,683 B2 offenbart ein Kartuschensystem, das zur Beimischen einer geringen Menge zu einer Hauptkomponente geeignet ist. Bei dem Kartuschensystem ist neben einer Kartusche eine zweite kleinere Kartusche angeordnet, wobei bei einem Vortrieb eines Austragskolbens in der größeren Kartusche auch ein Austragskolben in der kleineren Kartusche über ein gemeinsames Verbindungselement angetrieben wird. Das Verbindungselement schneidet sich dabei durch die Kartuschenwände, um die Gesamtlänge des Systems zu begrenzen. Nachteilig ist hieran, dass durch die Asymmetrie des Kartuschensystems dieses dazu neigt, dass die Austragskolben verkanten. Insbesondere bei hochviskosen pastösen Zementkomponenten zur Herstellung eines PMMA-Knochenzements wirkt sich dies besonders stark aus. Dadurch erfordert das Kartuschensystem, wenn es mit derartig zähen Zementkomponenten genutzt werden soll, beim Auspressen und Mischen einen derartig hohen Kraftauswand, dass dieser nicht mit herkömmlichen manuell angetriebenen Austragsvorrichtungen geleistet werden kann.

Aus der EP 0 119 847 A2 ist ein Kartuschensystem bekannt, bei dem eine axial angeordnete Trennwand den Innenraum der Kartusche in zwei Hohlräume für z.B. die miteinander zu vermischenden, viskosen Komponenten eines Zements teilt, und zwei miteinander, mit einem Keil, und mit einer Schneide verbundene Austragskolben in den Hohlräumen verschiebbar angeordnet sind, derart, dass beim Vortreiben der Austragskolben die Schneide die Trennwand aufschneidet und der Keil die aufgeschnittene Teile der Trennwand in Richtung der Innenwand der Kartusche drückt. Die DE 25 21 392 A1 offenbart eine Kartusche mit einer Scheidewand zur Bildung von zwei Kammern und mit einem Kolben, der zum Schneiden der Trennwand und zum Austragen des Inhalts der Kammern geeignet ist.

Ein koaxiales Kartuschensystem, das ein spezielles Kolbensystem enthält, wird im Patent EP1 392 450 B1 beschrieben. Das Kartuschensystem findet Anwendung in der Baustoffchemie für die Lagerung und Vermischung von pastenförmigen Zweikomponentendichtungsmassen. Das dort offenbarte Kolbensystem hat einen zylinderförmigen Austragskolben für die zentrale Kartusche und einen ringförmigen Austragskolben für die zweite, koaxial angeordnete Kartusche. Beide Austragskolben werden hinter den Dichtungsflächen über ein Auflageelement angetrieben, das auf der Rückseite mehrere Anlageflächen für den Stößel der Austragsvorrichtung besitzt. Das Auflageelement enthält bogenförmige Schneiden. Bei axialer Einwirkung des Stößels einer Auspressvorrichtung werden beide Kolben vorwärts in Richtung Kartuschenkopf bewegt. Dabei werden die in den koaxialen Kartuschen enthaltenen pastenförmigen Komponenten in Richtung Kartuschenkopf gedrückt. Gleichzeitig zerteilen zwei Schneiden die Wand der inneren koaxialen Kartusche in zwei Teile. Nachteilig ist an diesem System, dass zwangsläufig gleichzeitig zwei Schneidprozesse ablaufen. Das bedeutet, dass für beide Schneidprozesse Energie aufgebracht werden muss, die dadurch nicht zum eigentlichen Vortrieb der beiden pastöse Komponenten zur Verfügung steht. Die Vermischung von pastenförmigen Zementkomponenten für PMMA-Knochenzemente benötigt aufgrund der im Austragsrohr angeordneten statischen Mischer und der hohen Viskosität der Zementkomponenten sehr viel Vortriebsenergie, der bei größeren Kartuschen nicht mehr manuell, gefahrlos und nicht mit herkömmlichen Austragsvorrichtungen aufzubringen ist. Ein Verlust an Vortriebsenergie durch zwei gleichzeitig stattfindende Schneidprozesse kann daher insbesondere bei hoch viskosen pastenförmigen Komponenten problematisch sein. Zudem sind Koaxialkartuschen nicht ohne weiteres mit Zementkomponenten eines PMMA-Knochenzements zu befüllen. Insbesondere wenn nur geringe Mengen des PMMA-Knochenzements enthalten sein sollen, werden die freien Querschnitte der äußeren Koaxialkartusche so klein, dass sie nicht mit herkömmlichen Verfahren zu befüllen ist.

Die Aufgabe der Erfindung besteht also darin, die Nachteile des Stands der Technik zu überwinden. Insbesondere soll ein einfaches, kostengünstig zu fertigendes Lagerungs- und Mischsystem für pastenförmige Zweikomponenten-Polymethylmethacrylat-Knochenzemente und ein Verfahren zur Herstellung eines Zementteigs mit einem Lagerungs- und Mischsystem bereitgestellt werden, wobei das Lagerungs- und Mischsystem als einmalig zu verwendendes ready-to-use-System in einfachster Weise mit einer minimalen Anzahl von Montageschritten innerhalb weniger Sekunden einsatzbereit machbar sein soll und nach Verbindung mit manuell anzutreibenden medizinischen Austragsvorrichtungen beziehungsweise Applikatoren sofort nach Beginn der manueller Betätigung der Austragsvorrichtung einen homogen gemischten Zementteig erzeugt und an der Austragsöffnung eines Austragsrohrs austrägt. Die in den OPs bisher für die konventionellen Polymethylmethacrylat-Knochenzemente genutzten manuell zu bedienenden Austragsvorrichtungen mit jeweils einer Schubstange und gegebenenfalls einem Teller sollen für den Austrag des Zweikomponenten-Polymethylmethacrylat-Knochenzements beziehungsweise des Zementteigs mit dem zu entwickelnden Lagerungs- und Mischsystem genutzt werden können. Dadurch soll die Beschaffung von speziellen Austragsvorrichtungen für den Austrag von pastenförmigen Zweikomponenten-Polymethylmethacrylat-Knochenzementen vermieden werden.

Bevorzugt soll das zu entwickelnde Lagerungs- und Mischsystem keine zwei miteinander verbundenen synchron vorzutreibende Schubstangen beziehungsweise Stößel erfordern, damit die gesamte Vorrichtung nicht wesentlich länger und größer wird als die bisher für die konventionellen Pulver-Flüssigkeits-Polymethylmethacrylat-Knochenzemente üblichen Mischsysteme und Vakuummischsysteme. Es soll eine einfache Lösung gefunden werden, die es erlaubt, möglichst mit nur einer Schubstange beziehungsweise Stößel und gegebenenfalls einem daran befestigten Teller zwei pastenförmige Zementkomponenten synchron und manuell aus der Vorrichtung auszutreiben. Die pastenförmigen Zementkomponenten des Knochenzements sollen getrennt innerhalb des Lagerungs- und Mischsystems sicher gelagert werden können. Zur Anwendung sollen beide pastenförmigen Zementkomponenten sicher zusammengeführt werden können. Das Lagerungs- und Mischsystem soll auch ein geringes Volumen des homogen gemischten Zementteigs von ca. 50 ml beziehungsweise maximal 70 ml austragen können, ohne dass größere Restmengen (mehr als 15 ml) in dem System verbleiben und aufwendig entsorgt werden müssen. Größere Volumina des Zementteigs werden nicht angestrebt. Die genannten geringen Mengen sind nämlich für viele Anwendungen ausreichend, wie beispielsweise Operationen (OPs) im Bereich des Knies.

Der Übergang von der Kartusche zum Austragsrohr soll konstruktiv möglichst so gestaltet sein, dass der Strömungswiderstand der pastenförmigen Zementkomponenten beim Auspressen möglichst niedrig ist. Als Zementkomponenten müssen pastenförmige Zementkomponenten verwendet werden, die unmittelbar nach dem Auspressen anwendbar sind, bei denen also keine Zeit zum Anquellen des PMMA-Knochenzements notwendig ist. Die Vorrichtung soll so beschaffen sein, dass eine Verwechselung der relevanten Montageschritte durch den Anwender konstruktiv soweit wie möglich ausgeschlossen ist und das Lagerungs- und Mischsystem auch von weitgehend ungeschultem Personal durchführbar ist. Weiterhin soll ein Verfahren zum Vermischen der pastenförmigen Zementkomponenten und zum Austragen des homogen gemischten Zementteigs bereitgestellt werden.

Die Aufgaben der Erfindung werden gelöst durch ein Lagerungs- und Mischsystem für pastenförmige Zweikomponenten-Polymethylmethacrylat-Knochenzemente, das Lagerungs- und Mischsystem aufweisend
a) eine röhrenförmige Kartusche mit einem zylindrischen Innenraum,
b) einen Kartuschenkopf, der ein Ende der röhrenförmigen Kartusche verschließt,
c) eine axial in dem zylindrischen Innenraum der Kartusche angeordnete Trennwand, wobei die Trennwand mit der Mantelfläche des zylindrischen Innenraums der Kartusche verbunden ist und wobei die Trennwand den durch den Kartuschenkopf begrenzten zylindrischen Innenraum der Kartusche in zwei räumlich voneinander getrennte Hohlräume teilt, wobei in dem ersten Hohlraum eine erste pastenförmige Zementkomponente enthalten ist und in dem separaten zweiten Hohlraum eine zweite pastenförmige Zementkomponente enthalten ist,
d) zwei axial in beiden Hohlräumen der Kartusche verschiebbar angeordnete Austragskolben, wobei die Austragskolben die beiden Hohlräume auf der dem Kartuschenkopf gegenüberliegenden Seite der Hohlräume abschließen,
e) wobei die Austragskolben an der dem Kartuschenkopf gegenüberliegenden Rückseite über ein Verbindungsmittel miteinander verbunden sind, wobei an dem Verbindungsmittel ein Keil oder Kegel mit einer Schneide an der zum Kartuschenkopf weisenden Vorderseite des Keils oder Kegels angeordnet ist, so dass beim Vortreiben der Austragskolben in den Hohlräumen in Richtung des Kartuschenkopfs die Schneide die Trennwand aufschneidet und der Keil oder Kegel die aufgeschnittene Teile der Trennwand in Richtung der Innenwand der Kartusche drückt.

Die beiden Teile der mit der Schneide aufgeschnittenen Trennwand bleiben also mit der Kartusche verbunden. Besonders bevorzugt verformt der Keil oder Kegel die beiden aufgeschnittenen Teile der Trennwand. Die Verformung bewirkt eine Verschiebung des Materials der Teile der aufgeschnittenen Trennwand in Richtung der Innenwand der Kartusche. Ganz besonders bevorzugt werden die Teile der aufgeschnittenen Teile der Kartusche in Richtung der Innenwand der Kartusche geklappt.

Der Keil oder Kegel weisen erfindungsgemäß bevorzugt an der der Schneide gegenüberliegenden Basis einen Durchmesser von wenigstens 1,5 mm auf, besonders bevorzugt von wenigstens 3 mm. Hierdurch wird sichergestellt, dass einem Stößel einer Austragsvorrichtung eine ausreichende Anlagefläche zum vortreiben des Verbindungsmittels und damit der Austragskolben geboten wird.

Die Schneide ist bevorzugt senkrecht zur Trennwand angeordnet und/oder die Schneide läuft senkrecht zur Trennwand durch die Trennwand hindurch. Des Weiteren kann vorgesehen sein, dass sich die Schneide entlang der Zylindersachse oder im Bereich der Zylinderachse des zylindrischen Innenraums bewegt, wenn das Verbindungsmittel in dem Innenraum der Kartusche axial bewegt wird.

Die beiden Hohlräume sind bevorzugt ebenfalls zylindrisch und haben besonders bevorzugt eine Grundfläche in Form eines Halbkreises oder in Form von Kreissegmenten.

Das Lager- und Mischsystem ist vorzugsweise für eine 1:1 Mischung der Zementkomponenten vorgesehen.

Es kann vorgesehen sein, dass die Trennwand zumindest halb so breit ist wie die Hälfte des maximalen Durchmessers senkrecht zur Achse der Kartusche. Es kann auch vorgesehen sein, dass die Trennwand eine ebene Fläche ist. Die Trennwand soll den zylindrischen Innenraum der Kartusche im Bereich der Achse des Innenraums der Kartusche in die zwei Hohlräume teilen, besonders bevorzugt in zwei gleich große Hohlräume teilen.

Mit einer alternativen Weiterbildung der vorliegenden Erfindung wird vorgeschlagen, dass die Austragskolben aus mehreren nicht miteinander verbundenen Teilen bestehen, vorzugsweise einem vorderen Teil und einem hinteren Teil, die miteinander verbindbar sind oder die aneinander anlegbar sind. Der vordere Teil wird dann einfach von dem hinteren Teil in den Hohlräumen vorangeschoben. Das Verbindungsmittel ist dabei mit den hinteren oder hintersten Teilen der Austragskolben verbunden oder verbindbar. Zur Stabilisierung der beiden Austragskolben und zur Stabilisierung der Bewegung der beiden Austragskolben in den Hohlräumen sind die beiden Austragskolben bevorzugt jeweils einteilig ausgeführt und fest mit dem Verbindungsmittel verbunden.

Bevorzugt kann vorgesehen sein, dass die Trennwand den Innenraum der Kartusche flüssigkeitsundurchlässig trennt und dadurch die beiden Hohlräume voneinander flüssigkeitsundurchlässig getrennt sind.

Hiermit wird sichergestellt, dass die beiden Zementkomponenten auch über längere Zeit in dem Lagerungs- und Mischsystem beziehungsweise innerhalb der Kartusche gelagert werden können. Es soll dabei vermieden werden, dass die flüssige und sehr bewegliche Monomerkomponente in den benachbarten Hohlraum kriecht und mit der anderen Zementkomponente reagiert.

Gemäß einer bevorzugten Weiterbildung der Erfindung kann vorgesehen sein, dass die Trennwand mit der Mantelfläche des zylindrischen Innenraums der Kartusche entlang zwei Verbindunglinien verbunden ist, die die Mantelfläche begrenzen, wobei bevorzugt die Verbindungslinien gegenüberliegend zueinander angeordnet sind und besonders bevorzugt die Achse der Kartusche in der Trennwand liegt.

Hierdurch können ein gleichmäßiges Vortreiben der Austragskolben in den Hohlräumen und ein gleichmäßiges Aufschneiden der Trennwand mit gleichbleibender Kraft erfolgen.

Es kann auch vorgesehen sein, dass die von dem Kartuschenkopf abgewandte Seite des Keils oder Kegels eine Anlagefläche für einen Stößel einer Austragsvorrichtung bildet. Der Stößel der Austragsvorrichtung ist dazu bevorzugt in einer Richtung in der Fläche der Trennwand ausgerichtet, damit der Druck, der von dem Stößel auf die Schneide ausgeübt wird, derart auf die Schneide drückt, dass die Schneide durch die Trennwand schneidet und der Keil oder Kegel durch den Schnitt in der Trennwand getrieben werden kann. Dabei soll es möglichst nicht zu einem Verkanten des Kegels oder Keils kommen.

Des Weiteren kann auch vorgesehen sein, dass die Hohlräume einen halbkreisförmigen oder kreissegmentförmigen Querschnitt aufweisen und die Austragskolben einen dazu passenden Querschnitt aufweisen, so dass die Austragskolben die Hohlräume in jeder axialen Position in den Hohlräumen verschließen.

Hiermit wird eine besonders einfaches und kostengünstig zu realisierendes Lagerungs- und Mischsystem ermöglicht.

Gemäß einer bevorzugten Weiterentwicklung des erfindungsgemäßen Lagerungs- und Mischsystems kann vorgesehen sein, dass die Austragskolben über das Verbindungsmittel derart voneinander beabstandet sind, dass der zwischen den Austragskolben liegende Spalt kleiner oder gleich groß ist, wie die Stärke der Trennwand.

Hiermit wird sichergestellt, dass die Austragskolben stabil in den Hohlräumen laufen und dass die Trennwand gut von dem Keil oder Kegel aufgebogen werden kann.

Es kann auch vorgesehen sein, dass die Trennwand eine Stärke von maximal 1,5 mm hat, bevorzugt von maximal 1,0 mm, und/oder die Trennwand eine solche Stärke hat, dass die Trennwand mit der Schneide, auf die eine Vortriebskraft von 1 kN einwirkt, zu schneiden und von dem Keil oder Kegel aufzubiegen ist.

Hiermit wird sichergestellt, dass die Trennwand, wenn sie aus üblichen Kunststoffen hergestellt ist, problemlos mit manuell angetriebenen Austragsvorrichtungen aufgeschnitten werden kann, während die Zementkomponenten von den Austragskolben aus den Hohlräumen ausgepresst werden.

Ferner kann vorgesehen sein, dass das Lagerungs- und Mischsystem ein Austragsrohr aufweist, an dem ein Befestigungsmittel zur Befestigung des Austragsrohrs an der Kartusche vorgesehen ist, wobei vorzugsweise das Austragsrohr anstelle des Kartuschenkopfs an der Kartusche zu befestigen ist.

Das Befestigungsmittel ist bevorzugt ein Innengewinde, das auf ein Außengewinde an der Kartusche geschraubt werden kann. Besonders bevorzugt wird das Außengewinde an der Kartusche auch zur lösbaren Befestigung des Kartuschenkopfs an der Kartusche verwendet.

Im Austragsrohr ist erfindungsgemäß bevorzugt ein statischer Mischer angeordnet. Mit der Erfindung wird auch vorgeschlagen, dass im Austragsrohr ein statischer Mischer angeordnet ist und dass an der Basis des Austragsrohrs als Verbindungsmittel ein Innengewinde, ein Außengewinde, Elemente eines Bajonettverschlusses und/oder Rastelemente eines Rastverschlusses angebracht sind.

Hiermit kann das Austragsrohr dazu verwendet werden, die Zementkomponenten zu mischen und punktgenau zu applizieren. Insbesondere bei schwer zugänglichen Orten zur Applikation eines PMMA-Knochenzements ist ein längeres Austragsrohr vorteilhaft.

Als statische Mischer kommen alle allgemein bekannten statischen Mischer in Betracht. An der Basis des Austragsrohrs sind als Verbindungsmittel ein Innengewinde und/oder ein Außengewinde und/oder Elemente eines Bajonettverschlusses und/oder Rastelemente eines Rastverschlusses angebracht. Mit diesem Verbindungsmittel kann das Austragsrohr mit der Kartusche mechanisch stabil verbunden werden. Diese Verbindung muss stabil sein, damit der bei dem Herauspressen der pastenförmigen Zementkomponenten auftretende hohe Druck nicht zu einem Ablösen des Austragsrohrs von der Kartusche führt. Besonders vorteilhafte Verbindungsmittel sind dabei Gewinde und ganz besonders vorteilhaft sind Doppelgewinde.

Bei Lagerungs- und Mischsystemen mit Austragsrohr kann zudem vorgesehen sein, dass das Verhältnis des Durchmessers des Innenraums der Kartusche zum Innendurchmesser des Austragsrohrs kleiner als 5 zu 2 ist, wobei bevorzugt das Verhältnis des Durchmessers des Innenraums der Kartusche zum Innendurchmesser des Austragsrohrs kleiner oder gleich 2 zu 1 ist und ganz besonders bevorzugt das Verhältnis des Durchmessers des Innenraums der Kartusche zum Innendurchmesser des Austragsrohrs 8 zu 5 ist.

Hierdurch wird erreicht, dass während des Vortreibens der Austragskolben eine ausreichende Strömungsgeschwindigkeit des PMMA-Knochenzements an der Austragsöffnung des Austragsrohrs erreicht wird.

Gemäß einer bevorzugten Weiterbildung kann auch vorgesehen sein, dass der Durchmesser des Innenraums der Kartusche kleiner oder gleich 25 mm ist, wobei bevorzugt der Durchmesser des Innenraums der Kartusche kleiner oder gleich 20 mm ist.

Es kann bei Lagerungs- und Mischsystemen mit Austragsrohr auch vorgesehen sein, dass der Durchmesser des Innenraums der Kartusche kleiner oder gleich 25 mm ist und der Innendurchmesser des Austragsrohrs kleiner oder gleich 15 mm ist, wobei bevorzugt der Durchmesser des Innenraums der Kartusche kleiner oder gleich 20 mm ist und der Innendurchmesser des Austragsrohrs kleiner oder gleich 12 mm ist.

Durch den erfindungsgemäßen Aufbau der Kartusche beziehungsweise der Kartusche und des Austragsrohrs gelingt es, beide pastöse Zementkomponenten des PMMA-Knochenzements in einer einzigen Kartusche unterzubringen, die noch durch manuelle Krafteinwirkung auspressbar ist und die aber auch gleichzeitig noch mit herkömmlichen Techniken befüllt werden kann. Bei größeren Durchmessern reicht eine manuelle Krafteinwirkung nicht mehr ohne weiteres aus, um die zähflüssigen Zementkomponenten des Knochenzements aus der Kartusche zu pressen.

Es wird erfindungsgemäß zudem vorgeschlagen, dass die Kartusche einteilig mit der darin angeordneten Trennwand aufgebaut ist, vorzugsweise die Kartusche und die Trennwand als einteiliges Spritzgussteil aus Kunststoff gefertigt ist.

Hierdurch wird erreicht, dass die beiden Hohlräume dicht voneinander getrennt sind, so dass auch eine längerfristige Lagerung der Zementkomponenten möglich ist.

Des Weiteren kann vorgesehen sein, dass die Schneide aus einer Stahllegierung, Aluminiumlegierung, Zinklegierung, Keramik, einem Polyamid, glasfaserverstärktem Polyamid, Polyimid, Polyamid-co-imid, Polyetherketon oder Polysulfon besteht.

Diese Materialien sind gut zum Schneiden der Trennwand geeignet und kostengünstig zu verarbeiten.

Mit einer Weiterbildung der Erfindung wird vorgeschlagen, dass das Verhältnis des Innendurchmessers der Kartusche zum Abstand des Austragskolbens zum Kartuschenkopf kleiner oder gleich 1 zu 4 ist, wobei bevorzugt das Verhältnis des Innendurchmessers der Kartusche zum Abstand des Austragskolbens zum Kartuschenkopf kleiner oder gleich 1 zu 10 ist.

Gemäß einer Weiterbildung der erfindungsgemäßen Vorrichtung kann vorgesehen sein, dass in dem Kartuschenkopf zwei Durchführungen vorgesehen sind, die die beiden Hohlräume mit der Umgebung des Lagerungs- und Mischsystems verbinden, wobei in den Durchführungen jeweils ein lösbarer Stopfen angeordnet ist.

Hiermit wird ermöglicht, dass die Zementkomponenten durch die Durchführungen in die Hohlräume eingefüllt werden können. Bei einer Variante der vorliegenden Erfindung können die Zementkomponenten nach Entfernen der Stopfen auch wieder durch die Durchführungen ausgepresst werden.

Bei bevorzugten Lagerungs- und Mischsystemen kann auch vorgesehen sein, dass die Kartusche auf der dem Kartuschenkopf gegenüberliegenden Seite ein Befestigungselement für eine Austragsvorrichtung aufweist und auf der Seite des Kartuschenkopfs mindestens ein Befestigungsmittel aufweist, insbesondere ein Außengewinde, ein Innengewinde, mindestens ein Element eines Bajonettverschlusses und/oder ein Rastelement eines Rastverschlusses als Befestigungsmittel besitzt.

Hiermit wird der Anschluss der Kartusche an die Auspressvorrichtung an der Rückseite beziehungsweise an ein Austragsrohr und/oder einen Verschluss des Kartuschenkopfs an der Vorderseite erleichtert.

Die Austragskolben können zur Befüllung der Hohlräume mit den Zementkomponenten verwendet werden, wenn sie zunächst an dem Kartuschenkopf anliegen. Die Zementkomponenten werden in die Hohlräume eingepresst und dabei die Austragskolben in Richtung des Verbindungsmittels beziehungsweise der Rückseite der Kartusche gedrückt, ohne dass im Inneren der Hohlräume unerwünschte Lufteinschlüsse verbleiben, die beim Austreiben der Zementkomponenten mit den Austragskolben aus den Hohlräumen stören würden. Es können Rastmittel vorgesehen sein, die die Austragskolben mit dem Verbindungsmittel verbinden.

Ebenfalls kann vorgesehen sein, dass die Kartusche, der Kartuschenkopf, die Trennwand und die Austragskolben aus Kunststoff gefertigt sind, wobei als Kunststoffe Polyethylen-co-vinylalkohol (EVOH), Polybutylenterephthalat (PBT), Polyethylenterephthalat (PET) und Poly-methacrylsäuremethylester-co-acrylnitril bevorzugt werden.

Der Aufbau mit Kunststoffen ist kostengünstig und einfach umsetzbar. Die bevorzugten Kunststoffe sind aufgrund ihrer Resistenz gegenüber den in den Zementkomponenten enthaltenen Chemikalien besonders gut geeignet.

Des Weiteren kann vorgesehen sein, dass der Kartuschenkopf durch eine gummielastischen Platte realisiert ist, die mit einer Überwurfkappe an der Kartusche befestigt ist, wobei die Überwurfkappe eine Bewegung der gummielastischen Platte von der Kartusche weg mit Hilfe eines überragenden Rands blockiert, und wobei die gummielastische Platte an der den Austragskolben zugewandten Seite eine Aussparung zur Aufnahme der Längsseite der Trennwand besitzt, und wobei vorzugsweise durch diese Aufnahme in der gummielastischen Platte zwei Bereiche definiert sind, wobei in jedem Bereich eine Durchführung angeordnet ist, die durch einen Stopfen verschlossen ist.

Hiermit wird eine gute Dichtwirkung des Lagerungs- und Mischsystems erreicht. Die Teilung der gummielastischen Platte in zwei Bereiche ist nicht so verstehen, dass die gummielastische Platte zwei separate Teile aufweisen muss. Die beiden Bereiche können also zusammenhängend sein und durch eine einteilige gummielastische Platte realisiert sein.

Es kann dabei auch vorgesehen sein, dass der Kartuschenkopf zusätzlich mit einer Kunststoffplatte aufgebaut ist, wobei die Kunststoffplatte auf oder unter der gummielastischen Platte im Kartuschenkopf angeordnet ist. Die Kunststoffplatte dient der zusätzlichen Abdichtung und fördert die chemische Beständigkeit der damit begrenzten Hohlräume gegen die Zementkomponenten. Die Anordnung der zusätzlichen Kunststoffplatte führt zu einer verbesserten Diffusionsbarriere gegenüber dem in den Zementkomponenten enthaltenen Methylmethacrylat.

Ferner kann vorgesehen sein, dass eine Überwurfkappe als Verbindungselement zur Verbindung des Kartuschenkopfs mit der Kartusche vorgesehen ist, wobei die Überwurfkappe ein Innengewinde oder ein Außengewinde oder ein Bajonettverschluss oder Rastelemente aufweist.

Die Überwurfkappe ist bevorzugt eine Überwurfmutter und kann auf die Kartusche aufgeschraubt werden. Die Überwurfkappe kann als Teil des Kartuschenkopfs aufgefasst werden. Hierdurch kann der Kartuschenkopf stabil mit der Kartusche verbunden werden. Mit dem Verbindungselement kann die Überwurfkappe sicher mit der Kartusche verbunden werden. Eine Ablösung der Kartuschenkopfs von der Kartusche währender Lagerung und des Transports kann dadurch sicher verhindert werden.

Erfindungsgemäß kann vorgesehen sein, dass der Kartuschenkopf aus einer gummielastischen Platte und einer aus Kunststoff gefertigten Überwurfkappe gebildet ist, wobei die Überwurfkappe die gummielastische Platte nach oben durch einen überragenden Rand blockiert, wobei die gummielastische Platte an der Unterseite eine Aussparung zur Aufnahme der Längsseite der Trennwand besitzt, und wobei durch diese Aufnahme die gummielastische Platte in zwei halbkreisförmige Flächen geteilt ist, und wobei in jeder halbkreisförmigen Fläche eine Durchführung angeordnet ist, die durch einen Stopfen verschlossen ist. Die beiden Durchführungen sind zur Befüllung der beiden Hohlräume mit den pastenförmigen Zementkomponenten erforderlich. Die Überwurfkappe kann also auch als Teil des Kartuschenkopfs angenommen werden.

In einer Ausgestaltungsvariante ist die Stirnfläche der Kartusche als Kartuschenkopf ausgebildet, wobei die Unterseite eine Aussparung zur Aufnahme der Schmalseite der Trennwand besitzt, welche die Stirnfläche in zwei halbkreisförmige Flächen teilt, wobei in jeder halbkreisförmigen Fläche eine Durchführung angeordnet ist, die mit einem Stopfen verschlossen ist. Diese Durchführungen können zylinderförmige, halbkreisförmig, kreisabschnittförmig oder auch nierenförmig gestaltet sein. Entsprechend der Geometrie dieser Durchführungen sind dann auch die Stopfen zylinderförmig, halbkreisförmig, kreisabschnittförmig oder nierenförmig. Besonders vorteilhaft ist es, wenn die Stopfen an der Unterseite Rastelemente besitzen, damit ist eine Herauslösung der Stopfen aus dem Kartuschenkopf während der Lagerung und des Transports des Kartuschensystems wirksam verhindert.

Es kann erfindungsgemäß ferner vorgesehen sein, dass das Verbindungmittel derart gestaltet ist, dass die beiden aufgeschnittenen Teile der Trennwand mit der Innenwand der Kartusche verbunden bleiben, wenn das Verbindungsmittel in dem Innenraum der Kartusche in Richtung des Kartuschenkopfs vorgetrieben wird.

Hierdurch wird erreicht, dass die beiden aufgetrennten Teile der Trennwand an definierten Positionen im Innenraum der Kartusche befinden und es so zu keiner Blockade des Vortriebs kommt. Es ist auf diese Weise nicht möglich, dass sich die auftrennten Teile der Trennwand im Innenraum der Kartusche falten können, beziehungsweise Teile der Trennwand senkrecht zur Zylinderachse und damit senkrecht zum Vortrieb Falten bilden können, die eine Bewegung des Verbindungsmittels oder eines Stößels oder Tellers einer Austragsvorrichtung behindern könnten.

Die Schneide ist also erfindungsgemäß bevorzugt nicht dicht an einer Innenwand der Kartusche angeordnet. Es sind erfindungsgemäß bevorzugt keine Trennmittel oder Schneiden zum Auftrennen der Trennwand an den außen im Bereich der Innenwand der Kartusche liegenden Bereichen des Verbindungsmittels vorgesehen.

Die der vorliegenden Erfindung zugrundeliegenden Aufgaben werden auch gelöst durch ein Verfahren zur Vermischung von pastenförmigen Zementkomponenten eines pastenförmigen Zementteigs, insbesondere eines Polymethylmethacrylat-Knochenzements, mit einem solchen Lagerungs- und Mischsystem, gekennzeichnet durch die folgenden nacheinander ablaufenden Schritte,
a) Entfernen des Kartuschenkopfs von der Kartusche oder Entfernen von zumindest zwei Stopfen aus zumindest zwei Durchführungen im Kartuschenkopf, so dass die Kartusche geöffnet wird,
b) Aufsetzen und Verbinden eines Austragsrohrs mit der geöffneten Kartusche, wobei das Austragsrohr einen Mischer enthält,
c) Einsetzen der Kartusche in eine manuell zu bedienende Austragsvorrichtung, die Austragsvorrichtung aufweisend einen axial vortreibbaren Stößel zum Vortreiben der Austragskolben in den Hohlräumen der Kartusche, und
d) Auspressen der pastenförmigen Zementkomponenten mit Hilfe der Austragsvorrichtung durch axiales Vortreiben der Austragskolben mit dem Stößel, wodurch die Zementkomponenten in das Austragsrohr gedrückt werden, wobei die beiden Zementkomponenten durch den Mischer im Austragsrohr zu dem pastenförmigen Zementteig gemischt werden und der gemischte Zementteig aus einer Austragsöffnung des Austragsrohrs ausfließt, wobei synchron zur Bewegung der Austragskolben die Trennwand mit der Schneide in Längsrichtung der Kartusche aufgeschnitten wird und mit dem Keil oder Kegel die beiden aufgeschnittenen Teile der Trennwand in Richtung der Innenwand der Kartusche zumindest derart weit nach außen gedrückt werden, dass eine weitere Bewegung des Stößels der Austragsvorrichtung nicht durch die Teile der aufgeschnittenen Trennwand verhindert oder behindert wird.

Die Schritte laufen bevorzugt chronologisch nacheinander ab. Die Austragsvorrichtung wird vorzugsweise manuell angetrieben.

An dem in Richtung der Kartusche weisenden Ende des Stößels der Austragsvorrichtung kann ein Teller angeordnet sein, mit dem auf die Austragskolben beziehungsweise auf das Verbindungsmittel und den Keil oder Kegel gedrückt wird, um die Austragskolben in der Kartusche vorzutreiben. Die aufgeschnittenen Teile der Trennwand müssen dann derart weit nach außen an die Innenwand der Kartusche gedrückt werden, dass sie nicht in die Bewegung des Tellers eingreifen können.

Vor dem Entfernen des Kartuschenkopfs von der Kartusche oder dem Entfernen der zumindest zwei Stopfen aus den zumindest zwei Durchführungen im Kartuschenkopf ist die Kartusche beziehungsweise die Hohlräume der Kartusche, in denen die Zementkomponenten lagern, verschlossen.

Bei erfindungsgemäßen Verfahren kann vorgesehen sein, dass die beiden aufgeschnittenen Teile der Trennwand mit der Innenwand der Kartusche verbunden bleiben.

Hiermit wird sichergestellt, dass keine losen Teile der aufgeschnittenen Trennwand eine weitere Bewegung des Stößels behindern können.

Ferner kann vorgesehen sein, dass zur Entfernung des Kartuschenkopfs von der Kartusche in Schritt a) ein Verbindungselement gelöst wird, das den Kartuschenkopf mit der Kartusche verbindet.

Hierdurch wird eine stabilere Verbindung zwischen dem Kartuschenkopf und der Kartusche erreicht. Zudem kann das Gegenstück an der Kartusche, das heißt ein Verbindungselement an der Kartusche auch zur Verbindung des Austragsrohrs verwendet werden.

Gemäß einer bevorzugten Weiterbildung kann vorgesehen sein, dass das Austragsrohr mit der Kartusche durch Verbindung des Verbindungselements des Austragsrohrs mit einem Verbindungselement der Kartusche verbunden wird.

Hierdurch kann sichergestellt werden, dass sich das Austragsrohr beim Auspressen des Zementteigs nicht von der Kartusche löst.

Auch kann vorgesehen sein, dass der Stößel der Austragsvorrichtung auf die von den Austragskolben abgewandte Seite des Keils oder Kegels drückt und die Austragskolben über den Keil oder Kegel und das Verbindungsmittel angetrieben werden.

Dadurch wird erreicht, dass die durch den Stößel verfügbare Kraft mit einem möglichst großen Anteil zum Antreiben der Zementkomponenten sowie zum Schneiden und Trennen der Trennwand genutzt werden kann. Es soll dadurch vermieden werden, dass ein zu großer Teil der Kraft in eine ungewollte Verformung der Kartusche oder eine störende Verkantung der Austragskolben fließt.

Des Weiteren kann vorgesehen sein, dass die von den Austragskolben abgewandte Seite des Keils oder Kegels eine Anlagefläche zum Anlegen der Vorderseite des Stößels oder eines daran angebrachten Tellers aufweist, die gleich groß oder größer ist als der Querschnitt des Stößels oder als die Auflagefläche des Tellers, wobei beim Vortreiben des Stößels der Querschnitt des Stößels oder die Auflagefläche des Tellers vollständig von der Anlagefläche abgedeckt wird oder vorzugsweise die Anlagefläche den Querschnitt des Stößels oder die Auflagefläche des Tellers überragt.

Hiermit wird erreicht, dass die Bewegung des Stößels nicht von den aufgeschnittenen Teilen der Trennwand behindert wird.

Schließlich wird im Rahmen der vorliegenden Erfindung auch vorgeschlagen, dass der Applikator manuell antreibbar ist oder durch Druckluft oder elektrisch antreibbar ist.

Manuell antreibbare Applikatoren sind erfindungsgemäß bevorzugt, da sie weder an eine Druckluftquelle oder eine Energiequelle angeschlossen werden müssen, noch eine solche enthalten müssen.

Der Erfindung liegt die überraschende Erkenntnis zugrunde, dass die Zementkomponenten in einer einzigen gemeinsamen Kartusche mit zylindrischem Innenraum gelagert werden können, wenn die Zementkomponenten in der Kartusche über eine Trennwand, die im Innenraum der Kartusche angeordnet ist, voneinander getrennt werden, wobei beide Austragskolben über ein gemeinsames Verbindungsmittel ausgetrieben werden können, das die Trennwand aufschneidet und die aufgeschnittenen Teile der Trennwand aufbiegt, so dass eine weitere Bewegung eines antreibenden Stößels einer Austragsvorrichtung nicht behindert wird. Dadurch können handgetriebene Austragsvorrichtungen mit nur einem Stößel verwendet werden, da die notwendige Kraft aufgrund des erfindungsgemäßen Aufbaus dazu ausreicht, die Zementkomponenten anzutreiben und zu mischen sowie die Trennwand aufzuschneiden und zu verformen, beziehungsweise die notwendige Kraft vollständig in diese vier Vorgänge gesteckt werden kann. Daher ist der Kraftaufwand zum Vortreiben der Zementkomponenten, zum Mischen der Zementkomponenten und zum Aufschneiden und Trennen der Trennwand aufgrund des erfindungsgemäßen Aufbaus in der Summe nicht so groß, dass die Austragsvorrichtung insgesamt zu schwergängig wäre.

Ferner wurde überraschend gefunden, dass auf diese Weise eine schmale Kartusche mit nur einem einzigen Austragskolben zum Vortreiben der beiden Zementkomponenten verwendet werden kann. Dadurch kann die Kraft, die notwendig ist, um die Zementkomponenten zu mischen und auszutreiben, minimiert werden, so dass ein mit manueller Kraft anzutreibender Applikator zusammen mit dem Lagerungs- und Mischsystem eingesetzt werden kann, um die Zementkomponenten aus der Kartusche auszutreiben und miteinander zu mischen.

Die Erfindung basiert auf der Idee, zur Minimierung des Strömungswiderstands beim Austrag nur eine zylindrische Kartusche, anstelle von mehreren side-by-side-Kartuschen oder Koaxial-Kartuschen, für die separate Lagerung der beiden pastenförmigen Zementkomponenten einzusetzen. Zur Vermeidung von zwei Schubstangen und zwei Tellern zum Antreiben von zwei Austragskolben wird die zylindrische Kartusche dazu mit einer axialen und axial schneidbaren Trennwand ausgerüstet, die den Innenraum der Kartusche, der durch zwei Austragskolben und einen Kartuschenkopf begrenzt ist, in zwei Hohlräume teilt, in denen die beiden pastenförmigen Zementkomponenten separat gelagert werden können. Durch das Schneiden und Aufbiegen der Trennwand können auch kleinere Mengen des PMMA-Knochenzements verwendet werden und auch schmalere Kartuschen mit Innenräumen mit kleineren Innendurchmessern sind noch auspressbar.

In einer ersten Ausführungsform wird der Kartuschenkopf entfernt und das Austragsrohr, das einen statischen Mischer enthält, direkt mit einem Verbindungselement mit der Kartusche verbunden. Unmittelbar anschließend wird die Kartusche mit dem angeschlossenen Austragsrohr mit einer manuell zu betätigenden Austragsvorrichtung beziehungsweise einem manuell zu betätigenden Applikator verbunden und mit dem Austrag der Zementkomponenten beziehungsweise des Zementteigs begonnen. Diese Schritte erfordern einen Zeitaufwand von ungefähr 3 bis 8 Sekunden. Nach ungefähr 30 Sekunden kontinuierlicher Betätigung der Auspressvorrichtung beziehungsweise des Applikators ist bei einem gesamten Volumen beider pastenförmigen Zementkomponenten von maximal 60 ml der Austrag des Zementteigs also des gemischtem pastösen Zweikomponenten-PMMA-Knochenzements beendet.

In einer zweiten Ausführungsform werden nach Entfernung der Trennwand zwei Stopfen am Kartuschenkopf entfernt und das Austragsrohr mit statischem Mischer mit einem Verbindungselement mit der Kartusche verbunden.

Die Erfindung basiert weiterhin auf der Idee, dass ein im ersten Hohlraum axial beweglich angeordneter erster Austragskolben und im zweiten Hohlraum ein axial beweglicher zweiter Austragskolben angeordnet sind. Zwischen den Austragskolben befindet sich die Trennwand. An der Rückseite der Austragskolben sind diese über ein Verbindungselement miteinander verbunden. Die Vorderseite des Verbindungselements ist als senkrecht zur Trennwand stehende Schneide ausgebildet. Auf der Rückseite des Verbindungselements ist ein keilförmiger oder kegelförmiger Abstandhalter angeordnet, dessen Rückseite als Anlagefläche für den Stößel einer Austragsvorrichtung ausgebildet ist. Bei der Vorwärtsbewegung des Verbindungsmittels durch Einwirkung des Stößels in Richtung des Kartuschenkopfs wird durch die Schneide hinter den Austragskolben ein Längsschnitt in der Trennwand gesetzt und die beiden geschnittenen Teile der Trennwand bleiben jeweils mit der Innenwand des Hohlzylinders verbunden. Die beiden Hälften der in Längsrichtung getrennten Trennwand werden durch den keilförmigen oder kegelförmigen Abstandhalter nach außen zur Innenwand der Kartusche gedrückt. Dadurch kann der Stößel auf die Anlagefläche aufsetzen und über die gesamte Länge der Kartusche die Austragskolben unter Auspressung der pastenförmigen Zementkomponenten nach vorne drücken. Durch Setzen nur eines Schnitts an der Trennwand wird nur ein geringer Teil der Vortriebsenergie aufgewendet, so dass der Hauptanteil der Vortriebsenergie zum Vortreiben der Austragskolben und zum Vortreiben und Mischen der Zementkomponenten zur Verfügung steht.

Weiterhin basiert die Erfindung auf der Beobachtung, dass hochviskoser Zementteig aus zylinderförmigen Kartuschen durch ein Austragsrohr mit statischem Mischer mit handelsüblichen manuell anzutreibenden Auspressvorrichtungen beziehungsweise Applikatoren in akzeptabler Zeit und mit akzeptablem, da manuell aufbringbarem Kraftaufwand ausgetragen werden kann, wenn der Austragskolben an seiner Stirnseite einen Durchmesser von maximal 25 mm hat. Mit dem erfindungsgemäßen Aufbau wird ein Kartuschensystem bereitgestellt, das derartig kleine Durchmesser für die Anwendung hochviskoser Zementkomponenten realisieren kann. Dabei kann die Kartusche beziehungsweise können die Hohlräume dennoch ohne zu großen Aufwand mit den Zementkomponenten befüllt werden.

Ein beispielhaftes erfindungsgemäßes Lagerungs- und Mischsystem für pastenförmigen Zweikomponenten-Polymethylmethacrylat-Knochenzement ist zusammengesetzt aus
a) einem röhrenförmigen Hohlkörper als Kartusche,
b) einem Kartuschenkopf, der ein Ende des röhrenförmigen Hohlkörpers verschließt,
c) einer axial im röhrenförmigen Hohlkörper angeordneten Trennwand, wobei die Trennwand den Innenraum des Hohlkörpers in einen ersten Hohlraum und einen zweiten Hohlraum trennt,
d) einem halbkreisförmigen ersten Austragskolben, der axial beweglich im ersten Hohlraum angeordnet ist,
e) einem halbkreisförmigen zweiten Austragskolben, der axial beweglich im zweiten Hohlraum angeordnet ist,
f) einem Verbindungsmittel, das die Rückseite des ersten Austragskolbens mit der Rückseite des zweiten Austragskolbens verbindet, wobei das Verbindungsmittel die Austragskolben so beabstandet, dass der zwischen den Austragskolben liegende Spalt kleiner oder gleich der Dicke der Trennwand ist, wobei an der Vorderseite des Verbindungsmittels eine Schneide angeordnet ist, die senkrecht zum Spalt zwischen dem ersten Austragskolben und dem zweiten Austragskolben steht, wobei an der Rückseite des Verbindungsmittels ein keilförmiger oder kegelförmiger Abstandhalter angeordnet ist, dessen Rückseite als Anlagefläche für den Stößel der Austragsvorrichtung ausgebildet ist,
g) wobei die Dicke beziehungsweise Stärke der Trennwand kleiner oder gleich 1,5 mm ist, und
h) dass bei der Vorwärtsbewegung des Verbindungsmittels in Richtung des Kartuschenkopfs durch die Schneide hinter dem ersten Austragskolben und dem zweiten Austragskolben ein Längsschnitt in der Trennwand hinter den Austragskolben gesetzt wird und die beiden geschnittenen Teile der Trennwand jeweils mit der Innenwand des Hohlzylinders verbunden bleiben.

Vorteilhaft ist es, wenn der Holzylinder mit der Trennwand einteilig ausgebildet ist. Aufwendige Montageschritte zum Einsetzen einer separaten Trennwand in den Hohlzylinder entfallen dadurch. Bei der Verwendung von geeigneten Kunststoffen ist sichergestellt, dass die beiden Hohlräume flüssigkeitsundurchlässig getrennt sind. Grundsätzlich ist es aber auch möglich, eine separat hergestellte Trennwand in den Hohlzylinder einzusetzen. Die eingesetzte Trennwand kann dann durch mechanische Verklemmung oder durch Verschweißen oder durch Verklebung mit dem Hohlzylinder verbunden werden.

Die hochviskosen Zementkomponenten von pastenförmigen Polymethylmethacrylat-Knochenzementen enthalten das sehr flüchtige, radikalisch polymerisierbare Monomer Methylmethacrylat. Für eine Lagerung der Zementkomponenten ist es daher unerlässlich, dass die Innenwände der Kartusche, der Kartuschenkopf, die Austragskolben und die Trennwand aus Kunststoffen gebildet sind, die eine gute Diffusionsbarriere für Methylmethacrylat darstellen. Dabei sind Polyethylen-co-vinylalkohol (EVOH), Polybutylenterephthalat (PBT), Polyethylenterephthalat (PET) und Poly-methacrylsäuremethylester-co-acrylnitril als Kunststoffe bevorzugt. Es ist zusätzlich auch möglich, diffusionsdichte Metallschichten, Metall- oder Halbmetalloxidschichten oder Kunststoffschichten auf die nicht die Zementkomponenten berührenden Teile aufzubringen. Als Metallschichten kommen insbesondere Aluminiumschichten in Betracht. Als Halbmetalloxidschichten sind insbesondere Siliziumdioxid-Schichten geeignet.

Die Austragskolben oder alternativ dazu das Verbindungsmittel besitzen vorteilhaft Rastelemente, die mit Gegenrastelementen der Innenseite des Hohlzylinders der Kartusche verrasten können. Diese Rastelemente verhindern eine zum Kartuschenkopf entgegengesetzte Bewegung der Austragskolben. Die Verhinderung dieser Rückwärtsbewegung ist notwendig, wenn das mit den Zementkomponenten befüllte Kartuschensystem äußerlich mit Ethylenoxid sterilisiert wird. Bei der Sterilisation mit Ethylenoxid wird zur Entfernung der Luft aus der Sterilisationskammer Vakuum angelegt. Durch den Unterdruck kann es infolge der Verdampfung von Methylmethacrylat in den Zementkomponenten zu einem Überdruck in den Hohlräumen und damit zu einer unerwünschten Bewegung des Austragskolbens entgegengesetzt zum Kartuschenkopf kommen.

Besonders wichtig ist, dass der Innendurchmesser der Kartusche kleiner oder gleich 25 mm ist, damit der notwendige Auspressdruck zum Auspressen und Mischen der Zementkomponenten nicht zu hoch wird.

Ein beispielhaftes erfindungsgemäßes Verfahren zur Vermischung der pastenförmigen Zementkomponenten des pastenförmigen Polymethylmethacrylat-Knochenzements mit dem Kartuschensystem kann beispielsweise wie folgt umgesetzt werden:
a) Entfernung des Kartuschenkopfs von der Kartusche oder Entfernen von Stopfen aus den Durchführungen des Kartuschenkopfs,
b) Verbinden eines Austragsrohrs, das einen statischen Mischer enthält, mit der geöffneten Kartusche,
c) Verbinden der Kartusche mit einer manuell zu betätigenden Auspressvorrichtung beziehungsweise Austragsvorrichtung,
d) manuelle Betätigung der Auspressvorrichtung beziehungsweise Austragsvorrichtung, wobei der Stößel auf eine Anlagefläche des Verbindungsmittels drückt, wobei die pastenförmige erste Zementkomponente aus dem ersten Hohlraum durch den ersten Austragskolben und die pastenförmige zweite Zementkomponente aus dem zweiten Hohlraum durch den zweiten Austragskolben in das Austragsrohr und den statischen Mischer gepresst wird, wobei der gemischte Zementteig an der Öffnung des Austragsrohrs austritt, und
e) wobei synchron zur Vorwärtsbewegung der Austragskolben die Schneide die Trennwand hinter den Austragskolben in Längsrichtung aufschneidet, wobei die geschnittenen Teile der Trennwand mit der Innenwand des Hohlzylinders verbunden bleiben und hinter der Schneide durch den keilförmigen oder kegelförmigen Abstandhalter so nach außen in Richtung der Innenwand des Innenraums der Kartusche gedrückt werden, dass der Stößel der Austragsvorrichtung nicht in seiner Vorwärtsbewegung in Richtung Kartuschenkopf behindert wird.

In einer Variante dieses Verfahrens wird in den Schritten d) und e) anstelle der manuell angetriebenen Auspressvorrichtung eine durch Druckluft oder durch elektrischen Strom angetriebene Auspressvorrichtung verwendet.

Im Folgenden werden weitere Ausführungsbeispiele der Erfindung anhand von vierzehn schematisch dargestellten Figuren erläutert, ohne jedoch dabei die Erfindung zu beschränken. Dabei zeigt:
Figur 1: eine schematische perspektivische Querschnittansicht eines erfindungsgemäßen Lagerungs- und Mischsystems;
Figur 2: eine schematische perspektivische Querschnittansicht durch das erfindungsgemäße Lagerungs- und Mischsystem nach Figur 1 unmittelbar vor der Applikation des PMMA-Knochenzements, bei dem ein Austragsrohr an der Kartusche befestigt ist;
Figur 3: eine schematische perspektivische Explosionsdarstellung mit teilweise unterschiedlichen und alternativen Teilen von erfindungsgemäßen Lagerungs- und Mischsystemen, wobei links unten das zusammengesetzt System dargestellt ist;
Figur 4: eine vergrößerte schematische perspektivische Querschnittansicht des Kartuschenbodens des erfindungsgemäßen Lagerungs- und Mischsystems nach den Figuren 1 und 2;
Figuren 5 bis 8: schematische, teilweise geschnittene perspektivische Ansichten des Kartuschenbodens des erfindungsgemäßen Lagerungs- und Mischsystems nach den Figuren 1, 2 und 4 in unterschiedlichen Stadien während des Einsetzens des Verbindungsmittels und während des Vortreibens der Austragskolben;
Figur 9: eine schematische perspektivische Querschnittansicht auf den vorderen Teil des erfindungsgemäßen Lagerungs- und Mischsystems nach den Figuren 1, 2 und 4 bis 8;
Figuren 10 und 11: zwei schematische perspektivische Ansichten auf ein Verbindungsmittel für ein erfindungsgemäßes Lagerungs- und Mischsystem;
Figur 12: eine schematische Querschnittansicht durch das Verbindungsmittel nach den Figuren 10 und 11; und
Figuren 13 und 14: zwei schematische, teilweise geschnittene perspektivische Ansichten des Kartuschenbodens des erfindungsgemäßen Lagerungs- und Mischsystems nach den

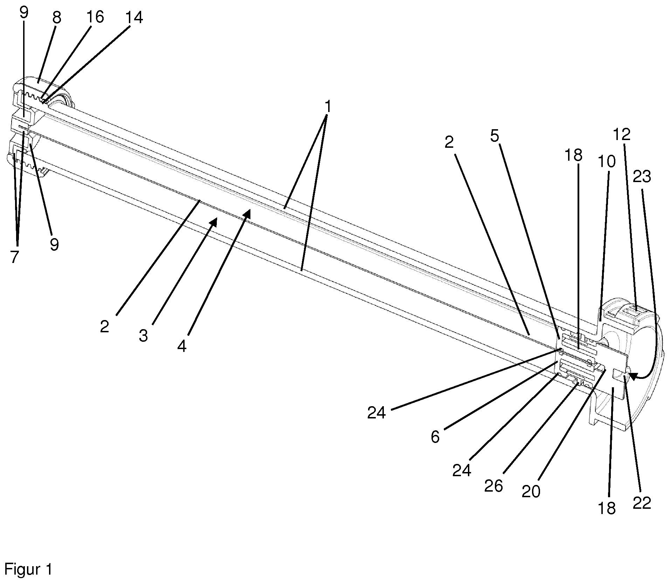

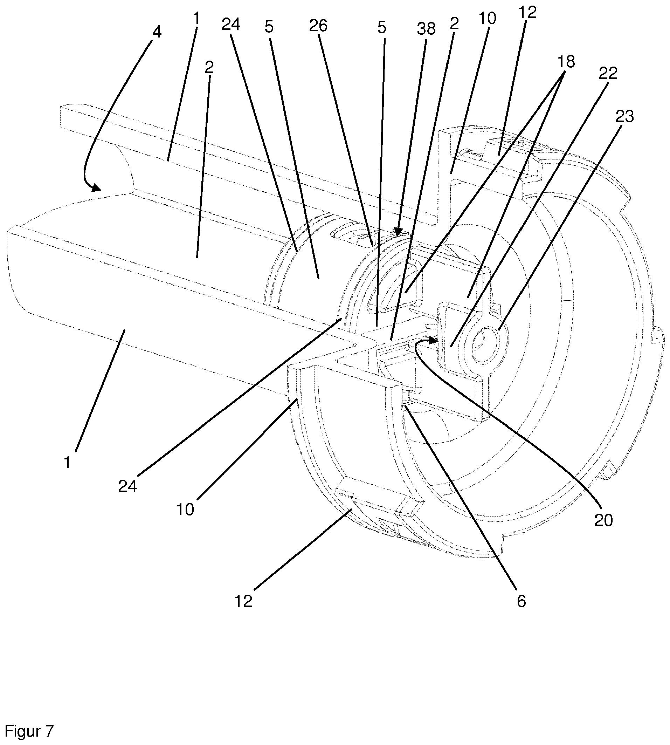

Figuren 1, 2 und 4 bis 9, das in eine Austragsvorrichtung zur Umsetzung eines erfindungsgemäßen Verfahrens eingesetzt ist.

In den Figuren werden der Einfachheit halber für gleiche und gleichartige Bauteile unterschiedlicher Ausführungsformen teilweise die gleichen Bezugszeichen verwendet.

Figur 1 zeigt eine schematische perspektivische Querschnittansicht eines erfindungsgemäßen Lagerungs- und Mischsystems. Das Lagerungs- und Mischsystem weist als zentralen Bestandteil eine zylindrische Kartusche 1 auf, bei der eine Trennwand 2 zwei gegenüberliegende Innenseiten der Innenwand der zylindrischen Kartusche 1 verbindet. Die Kartusche 1 und die Trennwand 2 sind einteilig als gemeinsames Spitzgussteil aufgebaut. Die Trennwand 2 trennt den Innenraum der Kartusche 1 in zwei separate und fluiddicht voneinander getrennte Hohlräume 3, 4, in denen die beiden pastösen Ausgangskomponenten eines PMMA-Knochenzements lagern.

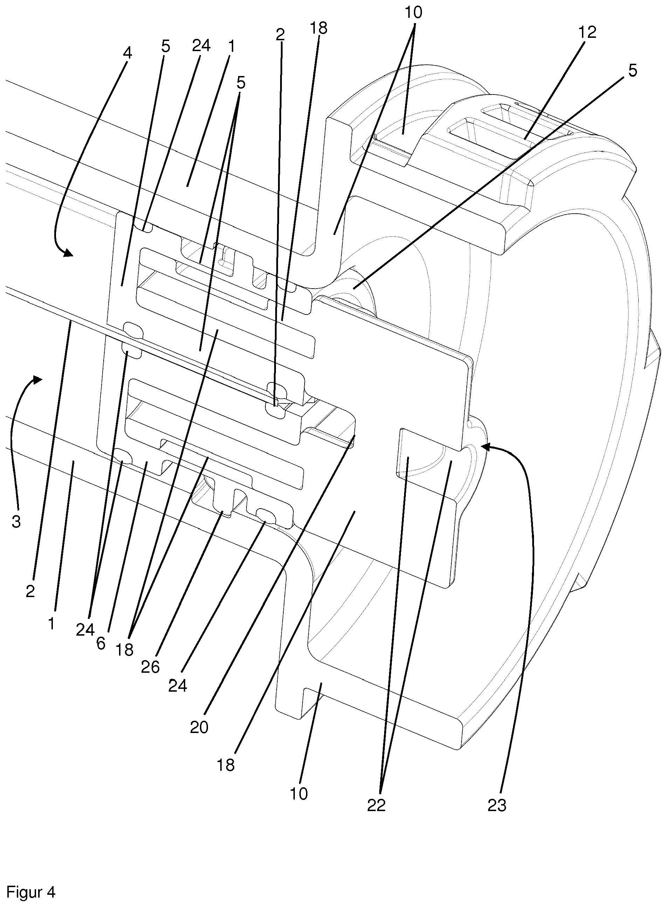

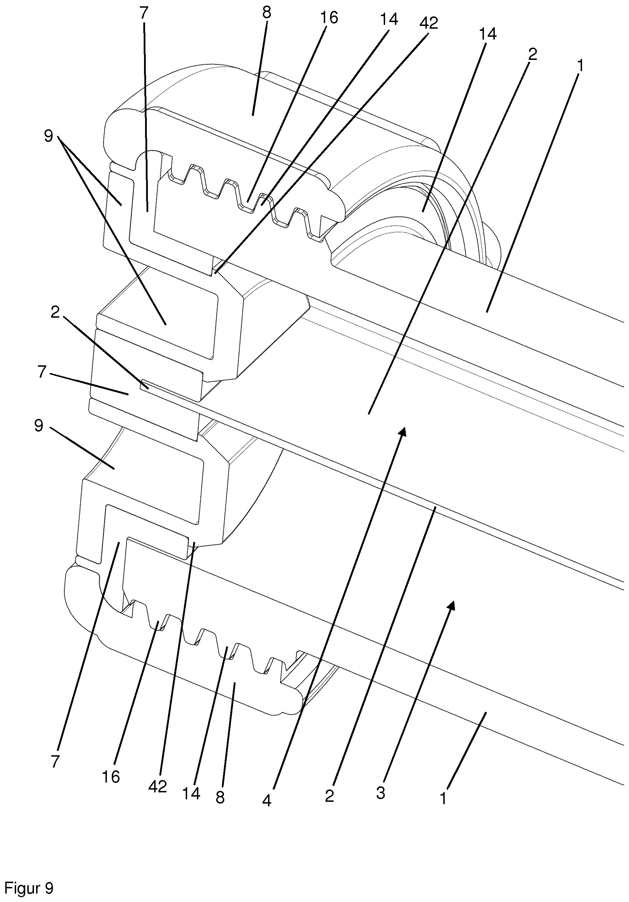

Auf der Rückseite (in Figur 1 rechts) sind die Hohlräume 3, 4 durch zwei Austragskolben 5, 6 begrenzt, wobei die Austragskolben 5, 6 axial in den beiden Hohlräumen 3, 4 verschiebbar gelagert sind. Figur 4 zeigt hierzu eine Detailansicht in Form einer vergrößerten schematischen perspektivischen Teilquerschnittansicht des Kartuschenbodens des erfindungsgemäßen Lagerungs- und Mischsystems nach Figur 1. An der den Austragskolben 5, 6 gegenüberliegenden Vorderseite des Lagerungs- und Mischsystems sind die beiden Hohlräume 3, 4 durch einen Kartuschenkopf 7 in Form einer gummielastischen Platte 7 verschlossen. Der Kartuschenkopf 7 ist mit einer Überwurfmutter 8 aus Kunststoff an der Vorderseite der Kartusche 1 befestigt. In dem Kartuschenkopf 7 sind zwei Durchführungen vorgesehen, die mit jeweils einem Stopfen 9 verschlossen sind. Durch die beiden Durchführungen sind die Hohlräume 3, 4 zugänglich, wenn die Stopfen 9 nicht darin eingesteckt sind. Figur 9 zeigt hierzu eine schematische perspektivische Querschnittansicht auf den vorderen Teil des erfindungsgemäßen Lagerungs- und Mischsystems als Detailansicht, wobei das Lagerungs- und Mischsystem in Figur 9 durch den Kartuschenkopf 7 und die Stopfen 9 darin wie in Figur 1 geschlossen ist.

An der Rückseite der Kartusche 1 beziehungsweise bodenseitig (in Figur 1 rechts) ist an der Kartusche 1 ein Anschluss 10 mit Befestigungselementen 12 angeordnet. Über den Anschluss 10 und die Befestigungselemente 12 kann die Kartusche 1 an eine Austragsvorrichtung beziehungsweise einen Applikator (in Figur 1 nicht gezeigt) angeschlossen werden. An der gegenüberliegenden Vorderseite (in Figur 1 links) der Kartusche 1 ist der Kartuschenkopf 7 mit der Überwurfmutter 8 befestigt, indem auf ein Außengewinde 14 an der Kartusche 1 ein Innengewinde 16 der Überwurfmutter 8 aufgeschraubt ist. Die gummielastische Platte 7 beziehungsweise der Kartuschenkopf 7 dichtet dabei die Hohlräume 3, 4 nach vorne ab.

Die Kartusche 1 hat einen Außendurchmesser von 22 mm, einen Innendurchmesser von 20 mm und eine Länge von etwa 18 cm.

Die beiden Austragskolben 5, 6 sind an deren Rückseite über ein Verbindungsmittel 18 miteinander verbunden. Das Verbindungsmittel 18 erstreckt sich dazu mit zwei in Richtung des Kartuschenkopfs 7 weisenden zylindersegmentförmigen Enden in passende Hohlräume in den Rückseiten der Austragskolben 5, 6 hinein. Eine in Richtung der Trennwand 2 weisende vordere Kante des Verbindungsmittels 18 ist als Klinge mit einer Schneide 20 ausgeführt, die sich in Richtung der Rückseite des Lagerungs- und Mischsystems über einen Keil 22 oder Kegel 22 beziehungsweise keilförmig und bereichsweise kegelförmig verbreitert. An der Rückseite des Keils 22 beziehungsweise Kegels 22 ist das Verbindungsmittel 18 als Anlagefläche 23 für einen Stößel einer Austragsvorrichtung (nicht gezeigt) ausgeformt.

Die Austragskolben 5, 6 sind über jeweils zwei umlaufende Dichtungen 24 aus Gummi gegen die Innenwand der Kartusche 1 und gegen die Trennwand 2 abgedichtet. Die Austragskolben 5, 6 sind mit jeweils einem lösbaren Rastelement 26 mit passenden Gegenrastelementen (in Form von zwei Vertiefungen) in der Innenwand der Kartusche 1 verbunden. Die Austragskolben 5, 6 können durch einen Druck von der Rückseite der Kartusche 1 (in Figur 1 von rechts) in Richtung der Vorderseite der Kartusche 1 (in Figur 1 links) also in Richtung des Kartuschenkopfs 7 gedrückt werden. Die Rastelemente 10 können durch einen bodenseitigen Druck auf die Austragskolben 5, 6, wenn der Kartuschenkopf 7 oder zumindest die Stopfen 9 entfernt sind, ohne weiteres gelöst werden und dienen vor allem dazu, dass die Austragskolben 5, 6 beim Befüllen des Innenraums der Kartusche 1 mit den Zementkomponenten nicht bodenseitig aus der Kartusche 1 herausgedrückt werden können, beziehungsweise nicht über die gewünschte, durch die Gegenrastelemente in der Innenwand der Kartusche 1 definierte Position hinaus in Richtung des Kartuschenbodens (in Figur 1 rechts) gedrückt werden können.

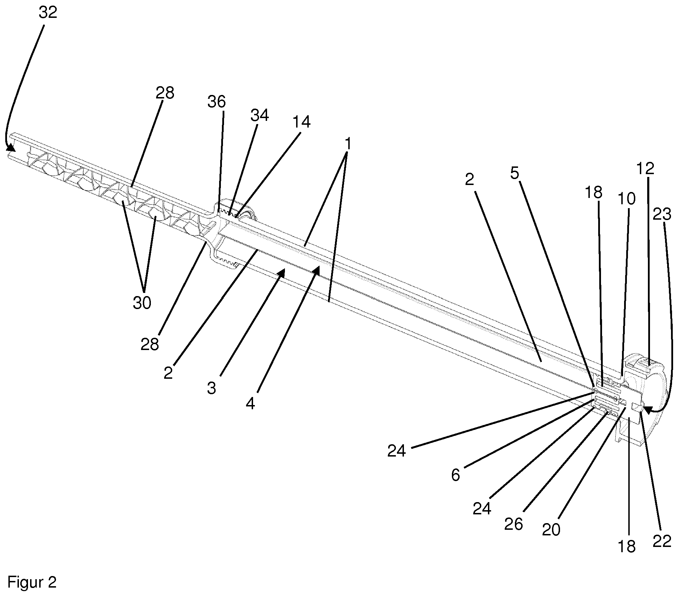

Figur 2 zeigt eine schematische perspektivische Querschnittansicht durch das erfindungsgemäße Lagerungs- und Mischsystem nach Figur 1, unmittelbar vor der Applikation des PMMA-Knochenzements, bei dem ein Austragsrohr 28 auf das Außengewinde 14 der Kartusche 1 aufgeschraubt ist. In dem Austragsrohr 28 ist ein statischer Mischer 30 mit einer Vielzahl von Wendungen und Mischelementen zur Vermischung der Zementkomponenten vorgesehen. Das Austragsrohr 28 kann auch noch länger sein als das in Figur 2 gezeigte Austragsrohr 28, um schwerer zugängliche Bereiche leichter erreichbar zu machen, wie dies beispielsweise bei Operationen an der Hüfte hilfreich sein kann. In Figur 2 sind alle Teile, bis auf den statischen Mischer 30 geschnitten dargestellt, während der statische Mischer 30 perspektivisch dargestellt aus der Schnittebene herausragt.

Die Zementkomponenten werden gemischt, indem sie durch das Austragsrohr 28 und damit durch den statischen Mischer 30 gedrückt werden. Der so hergestellte und gemischte Zementteig tritt über eine Austragsöffnung 32 an der Spitze des Austragsrohrs 28 aus. Das Austragsrohr 28 weist ein Innengewinde 34 auf, das auf das Außengewinde 14 der Kartusche 1 passt, so dass das Austragsrohr 28 stabil und fest mit der Kartusche 1 verbunden werden kann. Zwischen dem Austragsrohr 28 und der Vorderseite der Kartusche 1 ist ein Dichtungsring 36 angeordnet, damit die Zementkomponenten nicht zwischen dem Austragsrohr 28 und der Kartusche 1 austreten können. Über die Gewinde 14, 34 und den Dichtungsring 36 wird eine druckstabile und druckdichte Verbindung zwischen dem Austragsrohr 28 und der Kartusche 1 erreicht.

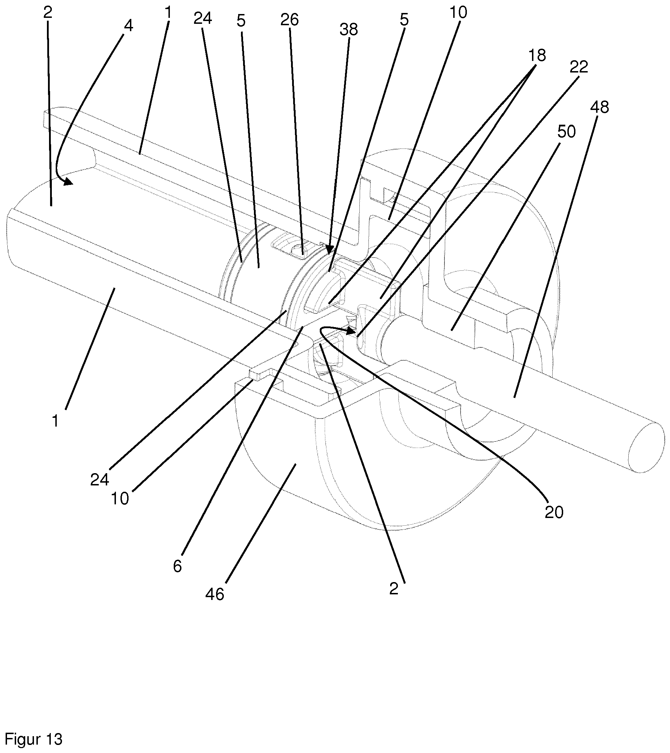

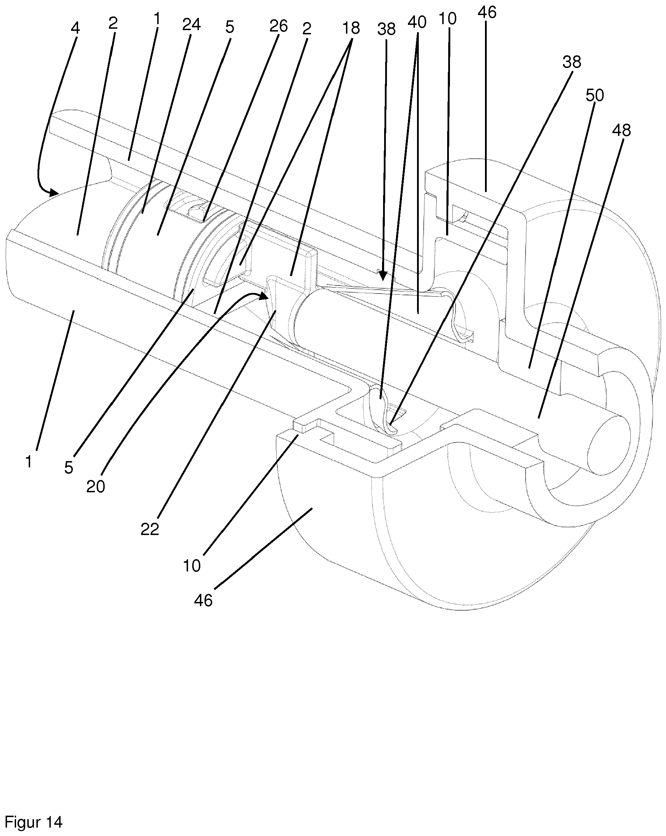

Der Vortrieb der Austragskolben 5, 6 wird über eine Austragsvorrichtung (in Figur 2 nicht gezeigt) erzeugt, die an den Anschluss 12 angeschlossen wird und mit der ein Stößel (siehe Figuren 13 und 14) beziehungsweise eine Schubstange der Austragsvorrichtung manuell in Richtung des Austragsrohrs 28 vorgetrieben werden kann. Der Stößel drückt dann auf die Anlagefläche 23, so dass einerseits die Austragskolben 5, 6 in Richtung des Austragsrohrs 28 vorgetrieben werden und andererseits die Schneide 20 in die Trennwand 2 getrieben wird und diese dabei aufschneidet. Der Druck des Stößels löst dabei die Rastelemente 10 und treibt die Austragskolben 5, 6 nach vorne. Die Austragskolben 5, 6 schließen dicht mit den Innenwänden der Kartusche 1 und der Trennwand 2 ab. Dadurch kann der Inhalt der Hohlräume 3, 4 der Kartusche 1, nämlich die zwei enthaltenen pastösen Zementkomponenten, nach vorne durch das Austragsrohr 8 ausgetrieben werden.

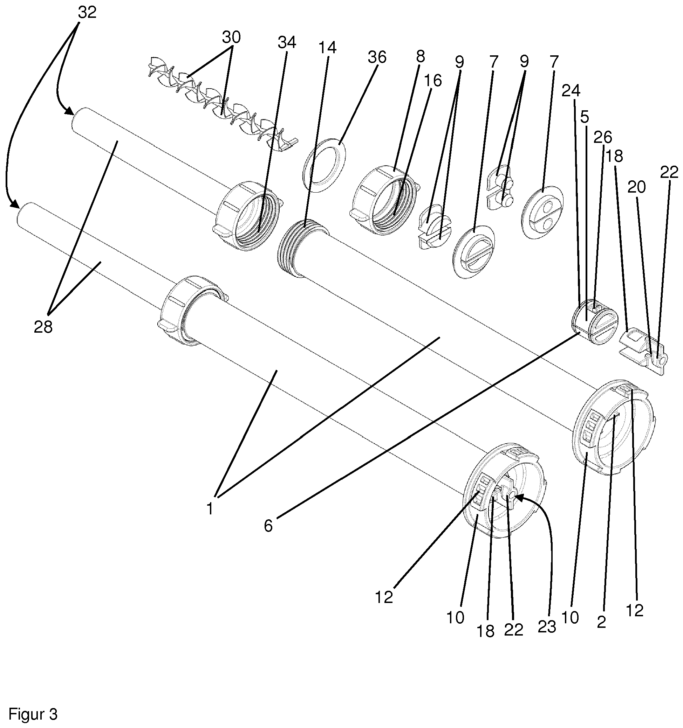

Figur 3 zeigt eine schematische perspektivische Explosionsdarstellung mit teilweise unterschiedlichen und alternativen Teilen von erfindungsgemäßen Lagerungs- und Mischsystemen. Der Aufbau der Lagerungs- und Mischsysteme ist analog dem zuvor beschriebenen Lagerungs- und Mischsystem. Links unten ist zudem ein zusammengesetzte erfindungsgemäßes Lagerungs- und Mischsystem in einer schematischen perspektivischen Ansicht gezeigt.

In Figur 3 sind zwei alternative Varianten der gummielastischen Platten 7 als Kartuschenköpfe 7 mit unterschiedlich geformten Durchführungen dargestellt, die mit unterschiedlichen Stopfen 9 geschlossen werden können. Die erste Variante, die bereits in Figur 1 dargestellt ist, ist in Figur 3 links unten relativ zu der zweiten Variante dargestellt.

In den beiden Durchführungen kann jeweils ein Stopfen 9 eingesteckt und gerastet werden. Die gummielastischen Platten 7 und die Stopfen 9 sind zum Aufbau von Kartuschenköpfen 7 erfindungsgemäßer Lagerungs- und Mischsysteme geeignet. Die gummielastischen Platten 7 unterscheiden sich in der Form ihrer Durchführungen und in der Form der Stopfen 9, die diese Durchführungen verschließen. Bei der ersten Variante (Variante links unten) sind die Durchführungen und die Stopfen 9 im Querschnitt halbkreisförmig beziehungsweise halbmondförmig. Bei der zweiten Variante (Variante rechts über der ersten Variante) sind die Durchführungen und die Stopfen 9 im Querschnitt kreisförmig. Bei der ersten Variante ist der freie Querschnitt zum Einfüllen der beiden Zementkomponenten größer als bei der zweiten Variante. Dafür ist bei der zweiten Variante die Geometrie an Einfüllrohre oder Spritzen (nicht gezeigt) angepasst, über die die Zementkomponenten in die Hohlräume 3, 4 eingefüllt werden, so dass die Einfüllrohre beziehungsweise Spritzen dicht mit den Durchführungen abschließen. Die Stopfen 9 können auch entfernt werden, um die Zementkomponenten wieder aus den Hohlräumen 3, 4 der Kartusche 1 auszutragen, wenn nicht der gesamte Kartuschenkopf 7 entfernt werden soll.

Bei den beiden in Figur 3 gezeigten Varianten können an den gummielastischen Platten 7 an den in den Innenraum der Kartusche 1 weisenden Seiten flache Kunststoffscheiben (nicht gezeigt) aufgesetzt sein. Diese Kunststoffscheiben dienen einerseits der Stabilisierung der Form der gummielastischen Platten 7 und andererseits der Verbesserung der chemischen Stabilität des Behältnisses beziehungsweise der Hohlräume 3, 4 für die Zementkomponenten.

Der Aufbau der rohrförmigen Kartusche 1 mit der Trennwand 2, dem Anschluss 10, den Befestigungselementen 12 und dem Außengewinde 14, ist bei allen Varianten gleich geformt. Auf das Außengewinde 14 der Kartusche 1 kann sowohl die Überwurfmutter 8 als auch das Austragsrohr 28 aufgeschraubt werden. Es sind auch Varianten denkbar, bei denen die gummielastische Platte 7 fest mit der Kartusche 1 verbunden ist oder auch nicht gummielastisch und/oder einteilig mit der Kartusche 1 ausgeführt ist. Dann wird keine Überwurfmutter 8 benötigt und das Austragsrohr 28 kann nach entfernen der Stopfen 9 einfach auf die Kartusche 1 aufgeschraubt werden. Die Zementkomponenten können dann einfach durch die Durchführungen in das Austragsrohr 28 gepresst werden und dort mit dem statischen Mischer 30 zu dem gewünschten Zementteig gemischt werden.

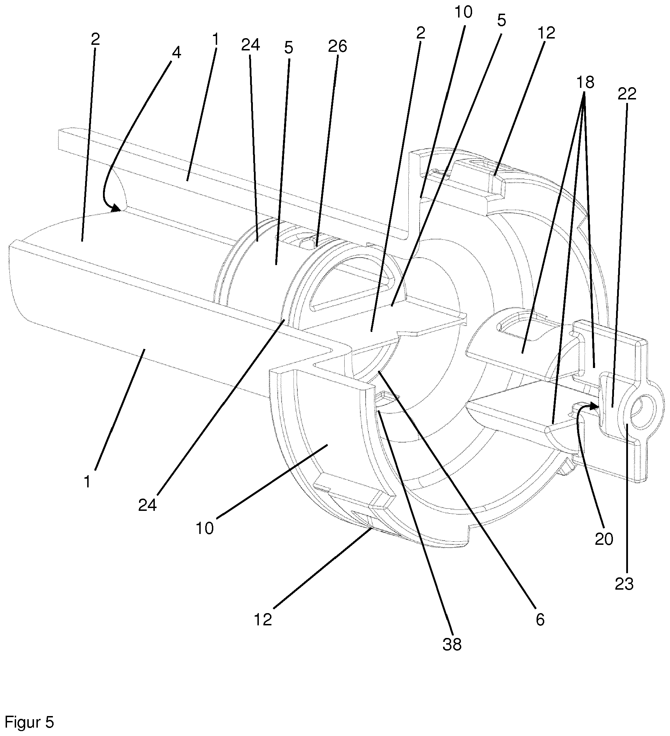

Die Figuren 5 bis 8 zeigen schematische, teilweise geschnittene perspektivische Ansichten des Kartuschenbodens des erfindungsgemäßen Lagerungs- und Mischsystems nach den Figuren 1, 2 und 4 in unterschiedlichen Stadien während des Vortreibens der Austragskolben 5, 6. In Figur 5 ist das Verbindungsmittel 18 noch nicht bodenseitig in die Kartusche 1 eingesteckt und die Austragskolben 5, 6 noch nicht in ihrer Endposition eingerastet. Die Rastmittel 26 der Austragskolben 5, 6 rasten nämlich in Vertiefungen 38 in der Innenwand der Kartusche 1 ein, wenn sie ausreichend tief in Richtung der Rückseite des Lagerungs- und Mischsystems in die Hohlräume 3, 4 der Kartusche 1 eingeschoben sind. Die Rastmittel 26 blockieren dabei eine weitere Bewegung der Austragskolben 5, 6 in Richtung der Rückseite des Lagerungs- und Mischsystems, während sie bei einer Bewegung in Richtung des Kartuschenkopfs 7 bewegbar sind. In der Trennwand 2 ist in Richtung des Verbindungsmittels 18 eine Kerbe vorgesehen, durch die die Schneide 20 leichter in den gewünschten Schnitt in der Trennwand 2 gleiten kann und der Beginn des Schnitts definiert ist.

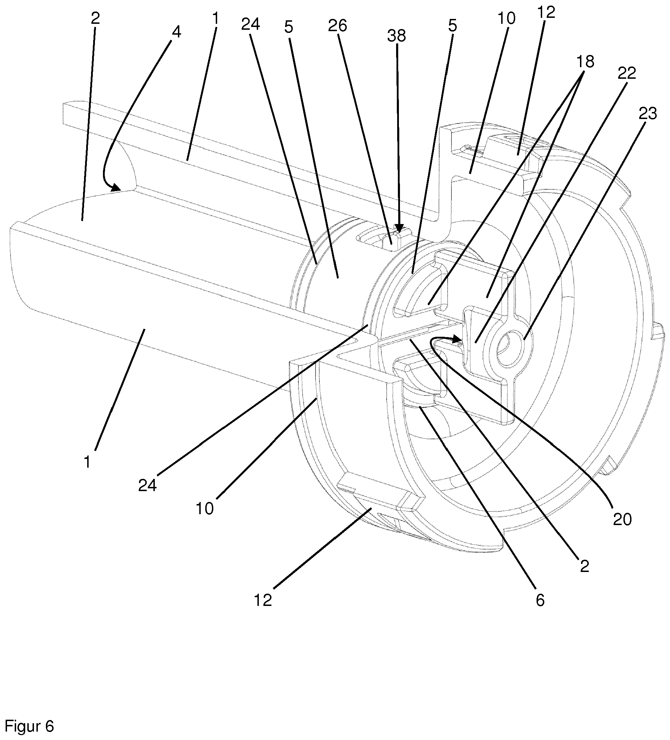

In der in Figur 6 gezeigten Position sind die Austragskolben 5, 6 mit den Rastmitteln 26 in die Vertiefungen 38 eingerastet und das Verbindungsmittel 18 ist bodenseitig in die Austragskolben 5, 6 eingesteckt und verbindet diese. Das Lagerungs- und Mischsystem ist dadurch einsatzbereit. Der Kartuschenkopf 7 kann getrennt werden oder die Stopfen 9 können aus den Durchführungen im Kartuschenkopf 9 entfernt werden und anstatt der Überwurfmutter 8 wird das Austragsrohr 28 auf die Kartusche 1 aufgeschraubt. In dieser Position kann eine Austragsvorrichtung mit dem Anschluss 10 beziehungsweise dem Befestigungsmittel 12 des Lagerungs- und Mischsystems verbunden werden.

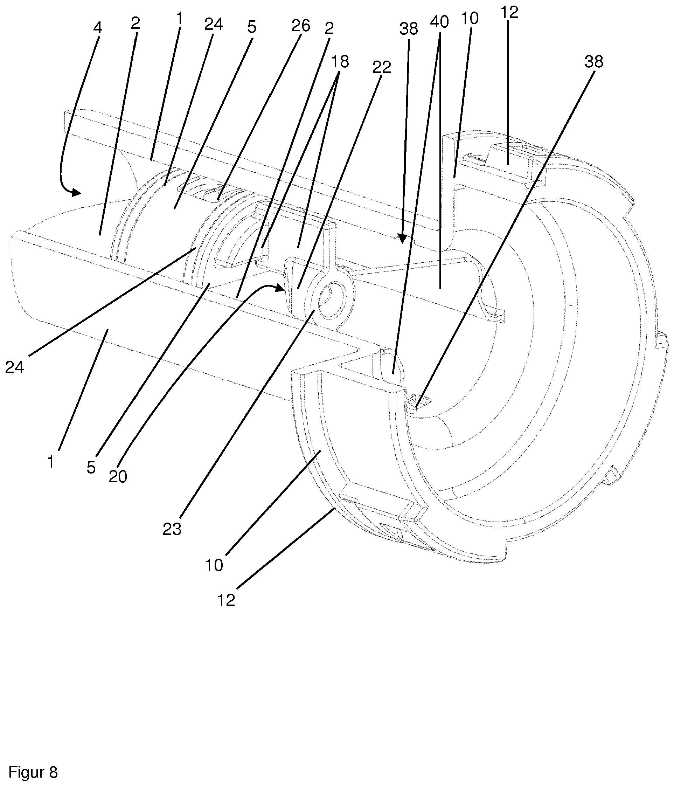

Das Verbindungsmittel 18 wird durch einen Druck auf die Anlagefläche 23 in Richtung des Kartuschenkopfs 7 vorgetrieben. Diese Situation ist zusammen mit den wesentlichen Teilen einer Austragsvorrichtung zum Vortreiben des Verbindungsmittels in Figur 13 gezeigt. Die Rastmittel 26 lösen sich aus den Vertiefungen 38 und die Austragskolben 5, 6 werden in den Hohlräumen 3, 4 nach vorne gedrückt. Dabei werden die beiden Zementkomponenten nach vorne in das Austragsrohr 28 gepresst und dort gemischt. Bei einem weiteren Vortreiben des Verbindungsmittels 18 werden nicht nur die Austragskolben 5, 6 weiter in den Hohlräumen 3, 4 der Kartusche 1 vorgetrieben, sondern die Schneide 20 greift auch in die Kerbe der Trennwand 2 und beginnt diese in axialer Richtung aufzuschneiden. Diese Situation ist in den Figuren 8 (ohne Austragsvorrichtung) und 14 (mit Teilen einer Austragsvorrichtung) gezeigt.

Durch weiteres Vortreiben des Verbindungsmittels 18 drückt der hinter der Schneide 20 angeordnete Keil 22 oder Kegel 22 die aufgeschnittenen Teile 40 der Trennwand 2, die noch immer mit der Innenwand der Kartusche 1 verbunden sind, auseinander und drückt diese in Richtung der Innenwand der Kartusche 1. Währenddessen werden die Zementkomponenten weiter aus den Hohlräumen 3, 4 in das Austragsrohr 28 gedrückt und dort gemischt. Schließlich tritt der fertig gemischte Zementteig durch die Austragsöffnung 32 aus dem Austragsrohr 28 aus und kann am gewünschten Ort appliziert werden.

Wie in Figur 9 zu erkennen ist, weisen die Stopfen 9 eine Rastung 42 in Form von Vorsprüngen auf, die über die Kante der Durchführungen in Richtung des Innenraums der Kartusche 1 in dem Kartuschenkopf 7 greifen und dadurch mit dem Kartuschenkopf 7 rasten. Durch die Rastung 42 wird erreicht, dass die Stopfen 9 sich nicht ungewollt aus dem Kartuschenkopf 7 lösen.

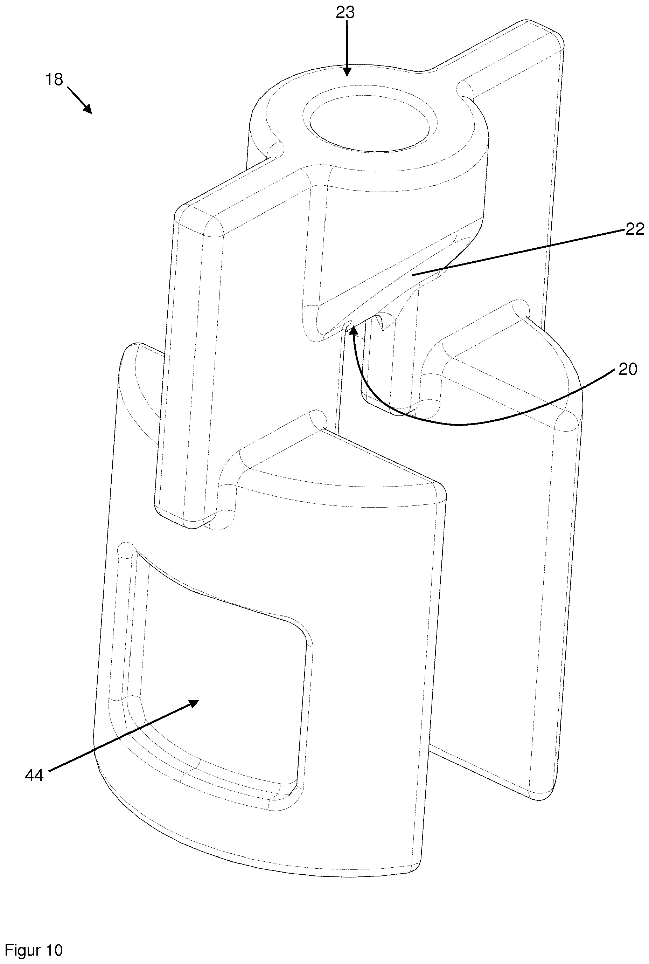

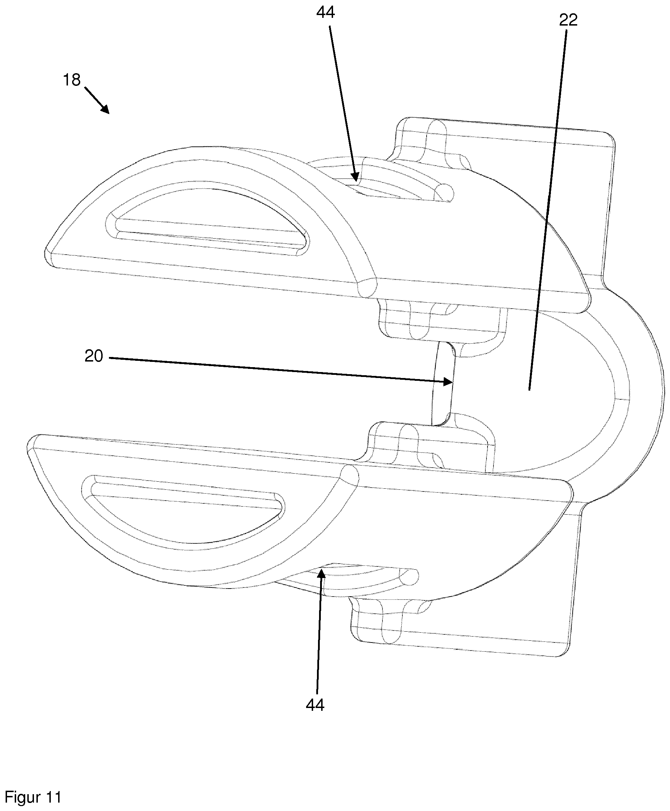

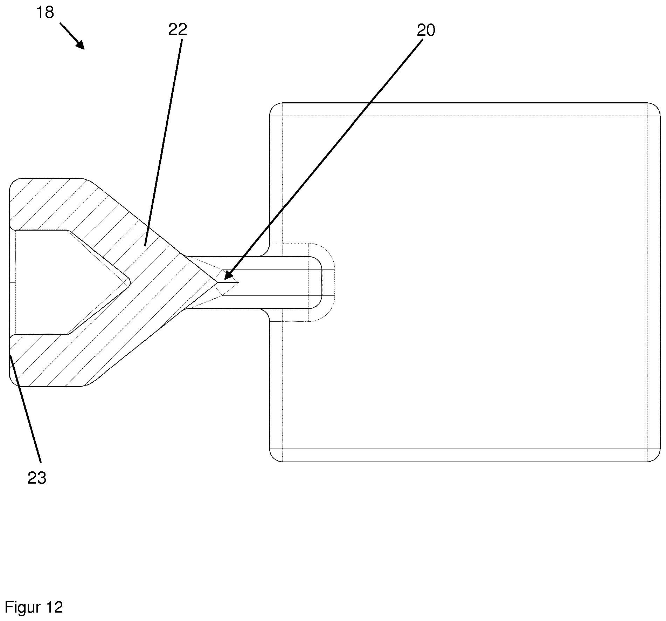

Figuren 10 und 11 zeigen zwei perspektivische Ansichten auf ein Verbindungsmittel 18 für ein erfindungsgemäßes Lagerungs- und Mischsystem und Figur 12 eine schematische Querschnittansicht durch das Verbindungsmittel 18 nach den Figuren 10 und 11. Das Verbindungsmittel 18 hat grob die Form eines Jochs und weist eine zweizählige Drehsymmetrie sowie eine Spiegelebene als Symmetrieebene auf, wobei die zweizählige Drehsymmetrieachse in der Spiegelebene liegt. Im eingebauten Zustand fällt die zweizählige Drehsymmetrieachse mit der Achse der Kartusche 1, also mit der Zylinderachse der Kartusche 1 zusammen.

Die dem Kartuschenkopf 7 zugewandte Seite des Verbindungsmittels 18 besteht aus zwei Zylindersegmenten, die in einer Ebene parallel zu deren Zylinderachse geschnitten sind, wobei an der Mantelfläche der Zylindersegmente zwei Vertiefungen 44 als Gegenrastungen 44 für ein je ein Rastmittel in den Austragskolben 5, 6 angeordnet sind. Die beiden Zylindersegmente des Verbindungsmittels 18 rasten also mit den Austragskolben 5, 6, wenn sie in die hierfür vorgesehenen Öffnungen auf der dem Kartuschenkopf 7 gegenüberliegenden Rückseite der Austragskolben 5, 6 eingesteckt werden.

Die beiden Zylindersegmente sind über eine Platte miteinander verbunden, in deren Mitte ein zentraler senkrechter Zylinder mit einer kreisförmigen Grundfläche 23 angeordnet ist, die die Anlagefläche 23 für einen Stößel 48 (siehe Figuren 13 und 14) einer Austragsvorrichtung bildet. Auf der gegenüberliegenden Seite des zentralen Zylinders ist die Grundfläche abgeschrägt und bildet den Keil 22 beziehungsweise Kegel 22. An der Spitze des Keils 22 oder Kegels 22 ist die Schneide 20 angeordnet, die den Spalt zwischen den beiden Zylindersegmenten überbrückt. Die beiden Zylindersegmente werden über die Platte und den zentralen senkrechten Zylinder in einem festen Abstand zueinander gehalten. Der Abstand ist so gewählt, dass die beiden Austragskolben 5, 6, wenn sie auf die Zylindersegmente des Verbindungsmittels 18 aufgesteckt sind, voneinander mit einem Abstand zueinander gehalten werden, der etwas kleiner oder höchstens genauso groß ist, wie die Stärke der Trennwand 2, also beispielsweise 1 mm. Die Schneide 20 kann ein Einsatz aus einem harten Metall oder einem harten Kunststoff sein, oder das gesamte Verbindungsmittel 18 kann aus einem solchen Material gefertigt sein, das hart genug sein muss, um die Trennwand 2 aufschneiden zu können, wenn das Verbindungsmittel 18 in der Kartusche 1 in Richtung des Kartuschenkopfs 7 vorgetrieben wird.

Die Figuren 13 und 14 zeigen zwei schematische, teilweise geschnittene perspektivische Ansichten des Kartuschenbodens des erfindungsgemäßen Lagerungs- und Mischsystems nach den Figuren 1, 2 und 4 bis 9, wobei das Lagerungs- und Mischsystem in eine Austragsvorrichtung zur Umsetzung eines erfindungsgemäßen Verfahrens eingesetzt ist. Von der Austragsvorrichtung ist in den Figuren 13 und 14 nur ein Anschluss 46 zum Verbinden mit den Befestigungsmitteln 12 des Lagerungs- und Mischsystems, ein Stößel 48 und eine Lagerung 50 für den Stößel 48 dargestellt. Diese Teile und die restlichen Bauteile der Austragsvorrichtung entsprechen denen üblicher manuell oder elektrisch oder pneumatisch angetriebener Austragsvorrichtungen. Die Austragsvorrichtung hat ein Fach zur Aufnahme des Lagerungs- und Mischsystems, wobei das Lagerungs- und Mischsystem zumindest an der Vorderseite im Bereich des Gewindes 14 und an der Rückseite am Anschluss 10 stabil gehalten wird. Der Anschluss 46 wird mit dem Befestigungsmittel 12 verbunden. Der Stößel 48, der als Schubstange 48 wirkt, kann gegen den Anschluss 46 der Austragsvorrichtung in Richtung durch den Anschluss 46 hindurch beziehungsweise in Richtung in die Kartusche 1 hinein bewegt beziehungsweise angetrieben werden, da er in der Lagerung 50 entlang seiner Längsachse beweglich gelagert ist. Dabei drückt die Spitze des Stößels 48 auf die Anlagefläche 23 des Verbindungselements 18. Dadurch wird das Verbindungselement 18 und die beiden Austragskolben 5, 6 in Richtung des Austragsrohrs 28 vorgetrieben.

Durch das Aufspreizen der beiden aufgeschnittenen Trennwandteile 40 mit Hilfe des Keils 22 oder Kegels 22 des Verbindungsmittels 18, werden die Trennwandteile 40 in Richtung der Innenwand des zylindrischen Innenraums der Kartusche 1 und weg von dem sich ins Innere der Kartusche 1 bewegenden Stößel 48 gedrückt. Dadurch können die beiden aufgeschnittenen Trennwandteile 40 die weitere Bewegung des Stößels 48 nicht behindern. Hierdurch gelingt es, dass trotz der hohen Zähigkeit der pastösen Zementkomponenten, trotz dem Strömungswiderstand, der durch den statischen Mischer 30 verursacht wird und trotz dem zum Schneiden der Trennwand 2 durch die Schneide 20 notwendigen Kraftaufwand beziehungsweise Energieaufwand der Widerstand gegen die Bewegung des Stößels 48 nicht so groß wird, dass die Kartusche 1 nicht mit herkömmlichen und auch manuell angetriebenen Austragsvorrichtungen auszupressen ist.

Kartusche 1 und Anschluss 10 sind bei allen Varianten bevorzugt einteilig ausgeführt und bestehen bevorzugt aus Kunststoff. Bis auf die Dichtungen 24 können alle Teile der Lagerungs- und Mischsysteme durch Spritzguss aus Kunststoff gefertigt werden. Die Dichtungen 24 bestehen bevorzugt aus Gummi. Auch die Platte 7 für den Kartuschenkopf 7 kann aus Gummi oder einem gummielastischen Werkstoff bestehen. Die Schneide 20 besteht bevorzugt aus einem Metall, einer Keramik, einer metallischen Legierung oder einem besonders harten Kunststoff. Theoretisch können auch die anderen Teile der Lagerungs- und Mischsysteme aus metallischen Werkstoffen gefertigt sein. Als Zementkomponenten werden bevorzugt pastöse Ausgangskomponenten eines PMMA-Knochenzements verwendet. Theoretisch können aber auch andere Zemente, wie beispielsweise Dentalzemente, Zweikomponenten-Klebstoffe oder andere Zweikomponenten-Systeme, die aus pastösen Ausgangskomponenten gemischt werden, mit einem erfindungsgemäßen Lagerungs- und Mischsystem gelagert und gemischt werden.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1: Kartusche
- 2: Trennwand
- 3: Hohlraum
- 4: Hohlraum
- 5: Austragskolben
- 6: Austragskolben
- 7: Kartuschenkopf
- 8: Überwurfmutter
- 9: Stopfen
- 10: Anschluss
- 12: Befestigungselement
- 14: Außengewinde
- 16: Innengewinde
- 18: Verbindungsmittel
- 20: Schneide
- 22: Keil / Kegel
- 23: Anlagefläche
- 24: Dichtung
- 26: Rastmittel
- 28: Austragsrohr
- 30: Statischer Mischer
- 32: Austragsöffnung
- 34: Innengewinde
- 36: Dichtung
- 38: Gegenrastmittel / Vertiefung
- 40: Aufgeschnittene Trennwand
- 42: Rastung
- 44: Vertiefung / Gegenrastung
- 46: Anschluss
- 48: Stößel / Schubstange
- 50: Lagerung

## Patentansprüche

1. Lagerungs- und Mischsystem für pastenförmige Zweikomponenten-Polymethylmethacrylat-Knochenzemente, das Lagerungs- und Mischsystem aufweisend
eine röhrenförmige Kartusche (1) mit einem zylindrischen Innenraum,
einen Kartuschenkopf (7), der ein Ende der röhrenförmigen Kartusche (1) verschließt, eine axial in dem zylindrischen Innenraum der Kartusche (1) angeordnete Trennwand (2), wobei die Trennwand (2) mit der Mantelfläche des zylindrischen Innenraums der Kartusche (1) verbunden ist und wobei die Trennwand (2) den durch den Kartuschenkopf (7) begrenzten zylindrischen Innenraum der Kartusche (1) in zwei räumlich voneinander getrennte Hohlräume (3, 4) teilt, wobei in dem ersten Hohlraum (3) eine erste pastenförmige Zementkomponente enthalten ist und in dem separaten zweiten Hohlraum (4) eine zweite pastenförmige Zementkomponente enthalten ist, zwei axial in beiden Hohlräumen (3, 4) der Kartusche (1) verschiebbar angeordnete Austragskolben (5, 6), wobei die Austragskolben (5, 6) die beiden Hohlräume (3, 4) auf der dem Kartuschenkopf (7) gegenüberliegenden Seite der Hohlräume (3, 4) abschließen,
wobei die Austragskolben (5, 6) an der dem Kartuschenkopf (7) gegenüberliegenden Rückseite über ein Verbindungsmittel (18) miteinander verbunden sind, wobei an dem Verbindungsmittel (18) ein Keil (22) oder Kegel (22) mit einer Schneide (20) an der zum Kartuschenkopf (7) weisenden Vorderseite des Keils (22) oder Kegels (22) angeordnet ist, so dass beim Vortreiben der Austragskolben (5, 6) in den Hohlräumen (3, 4) in Richtung des Kartuschenkopfs (7) die Schneide (20) die Trennwand (2) aufschneidet und der Keil (22) oder Kegel (22) die aufgeschnittene Teile (40) der Trennwand (2) in Richtung der Innenwand der Kartusche (1) drückt.

2. Lagerungs- und Mischsystem nach Anspruch 1, **dadurch gekennzeichnet, dass** die Trennwand (2) mit der Mantelfläche des zylindrischen Innenraums der Kartusche (1) entlang zwei Verbindunglinien verbunden ist, die die Mantelfläche begrenzen, wobei bevorzugt die Verbindungslinien gegenüberliegend zueinander angeordnet sind und besonders bevorzugt die Achse der Kartusche (1) in der Trennwand (2) liegt.

3. Lagerungs- und Mischsystem nach Anspruch 1 oder 2, **dadurch gekennzeichnet, dass**
die Hohlräume (3, 4) einen halbkreisförmigen oder kreissegmentförmigen Querschnitt aufweisen und die Austragskolben (5, 6) einen dazu passenden Querschnitt aufweisen, so dass die Austragskolben (5, 6) die Hohlräume (3, 4) in jeder axialen Position in den Hohlräumen (3, 4) verschließen.

4. Lagerungs- und Mischsystem nach einem der vorangehenden Ansprüche, **dadurch gekennzeichnet, dass**
die Austragskolben (5, 6) über das Verbindungsmittel (18) derart voneinander beabstandet sind, dass der zwischen den Austragskolben (5, 6) liegende Spalt kleiner oder gleich groß ist, wie die Stärke der Trennwand (2).

5. Lagerungs- und Mischsystem nach einem der vorangehenden Ansprüche, **dadurch gekennzeichnet, dass**
die Trennwand (2) eine Stärke von maximal 1,5 mm hat, bevorzugt von maximal 1,0 mm, und/oder die Trennwand (2) eine solche Stärke hat, dass die Trennwand (2) mit der Schneide (20), auf die eine Vortriebskraft von 3 kN einwirkt, zu schneiden und von dem Keil (22) oder Kegel (22) aufzubiegen ist.

6. Lagerungs- und Mischsystem nach einem der vorangehenden Ansprüche, **dadurch gekennzeichnet, dass**
in dem Kartuschenkopf (7) zwei Durchführungen vorgesehen sind, die die beiden Hohlräume (3, 4) mit der Umgebung des Lagerungs- und Mischsystems verbinden, wobei in den Durchführungen jeweils ein lösbarer Stopfen (9) angeordnet ist.

7. Lagerungs- und Mischsystem nach einem der vorangehenden Ansprüche, **dadurch gekennzeichnet, dass**
die Kartusche (1), der Kartuschenkopf (7), die Trennwand (2) und die Austragskolben (5, 6) aus Kunststoff gefertigt sind, wobei als Kunststoffe Polyethylen-co-vinylalkohol (EVOH), Polybutylenterephthalat (PBT), Polyethylenterephthalat (PET) und Poly-methacrylsäuremethylester-co-acrylnitril bevorzugt werden.

8. Lagerungs- und Mischsystem nach einem der vorangehenden Ansprüche, **dadurch gekennzeichnet, dass**
der Kartuschenkopf (7) durch eine gummielastischen Platte (7) realisiert ist, die mit einer Überwurfkappe (8) an der Kartusche (1) befestigt ist, wobei die Überwurfkappe (8) eine Bewegung der gummielastischen Platte (7) von der Kartusche (1) weg mit Hilfe eines überragenden Rands blockiert, und wobei die gummielastische Platte (7) an der den Austragskolben (5, 6) zugewandten Seite eine Aussparung zur Aufnahme der Längsseite der Trennwand (2) besitzt, und wobei vorzugsweise durch diese Aufnahme in der gummielastischen Platte (7) zwei Bereiche definiert sind, wobei in jedem Bereich eine Durchführung angeordnet ist, die durch einen Stopfen (9) verschlossen ist.

9. Lagerungs- und Mischsystem nach einem der vorangehenden Ansprüche, **dadurch gekennzeichnet, dass**
eine Überwurfkappe (8) als Verbindungselement zur Verbindung des Kartuschenkopfs (7) mit der Kartusche (1) vorgesehen ist, wobei die Überwurfkappe (8) ein Innengewinde (34) oder ein Außengewinde oder ein Bajonettverschluss oder Rastelemente aufweist.

10. Lagerungs- und Mischsystem nach einem der vorangehenden Ansprüche, **dadurch gekennzeichnet, dass**
das Verbindungmittel (18) derart gestaltet ist, dass die beiden aufgeschnittenen Teile (40) der Trennwand (2) mit der Innenwand der Kartusche (1) verbunden bleiben, wenn das Verbindungsmittel (18) in dem Innenraum der Kartusche (1) in Richtung des Kartuschenkopfs (7) vorgetrieben wird.

11. Verfahren zur Vermischung von pastenförmigen Zementkomponenten eines pastenförmigen Zementteigs, insbesondere eines Polymethylmethacrylat-Knochenzements, mit einem Lagerungs- und Mischsystem nach mindestens einem der vorangehenden Ansprüche, **gekennzeichnet durch** die folgenden nacheinander ablaufenden Schritte,
a) Entfernen des Kartuschenkopfs (7) von der Kartusche (1) oder Entfernen von zumindest zwei Stopfen (9) aus zumindest zwei Durchführungen im Kartuschenkopf (7), so dass die Kartusche (1) geöffnet wird,
b) Aufsetzen und Verbinden eines Austragsrohrs (28) mit der geöffneten Kartusche (1), wobei das Austragsrohr (28) einen Mischer (30) enthält,
c) Einsetzen der Kartusche (1) in eine manuell zu bedienende Austragsvorrichtung, die Austragsvorrichtung aufweisend einen axial vortreibbaren Stößel (48) zum Vortreiben der Austragskolben (5, 6) in den Hohlräumen (3, 4) der Kartusche (1),
d) Auspressen der pastenförmigen Zementkomponenten mit Hilfe der Austragsvorrichtung durch axiales Vortreiben der Austragskolben (5, 6) mit dem Stößel (48), wodurch die Zementkomponenten in das Austragsrohr (28) gedrückt werden, wobei die beiden Zementkomponenten durch den Mischer (30) im Austragsrohr (28) zu dem pastenförmigen Zementteig gemischt werden, wobei synchron zur Bewegung der Austragskolben (5, 6) die Trennwand (2) mit der Schneide (20) in Längsrichtung der Kartusche (1) aufgeschnitten wird und mit dem Keil (22) oder Kegel (22) die beiden aufgeschnittenen Teile (40) der Trennwand (2) in Richtung der Innenwand der Kartusche (1) zumindest derart weit nach außen gedrückt werden, dass eine weitere Bewegung des Stößels (48) der Austragsvorrichtung nicht durch die Teile (40) der aufgeschnittenen Trennwand (2) verhindert oder behindert wird.

12. Verfahren nach Anspruch 11, **dadurch gekennzeichnet, dass** die beiden aufgeschnittenen Teile (40) der Trennwand (2) mit der Innenwand der Kartusche (1) verbunden bleiben.

13. Verfahren nach einem der Ansprüche 11 oder 12, **dadurch gekennzeichnet, dass** das Austragsrohr (28) mit der Kartusche (1) durch Verbindung des Verbindungselements (34) des Austragsrohrs (28) mit einem Verbindungselement (14) der Kartusche (1) verbunden wird.

14. Verfahren nach einem der Ansprüche 11 bis 13, **dadurch gekennzeichnet, dass** der Stößel (48) der Austragsvorrichtung auf die von den Austragskolben (5, 6) abgewandte Seite (23) des Keils (22) oder Kegels (22) drückt und die Austragskolben (5, 6) über den Keil (22) oder Kegel (22) und das Verbindungsmittel (18) angetrieben werden.

15. Verfahren nach einem der Ansprüche 11 bis 14, **dadurch gekennzeichnet, dass** die von den Austragskolben (5, 6) abgewandte Seite (23) des Keils (22) oder Kegels (22) eine Anlagefläche (23) zum Anlegen der Vorderseite des Stößels (48) oder eines daran angebrachten Tellers aufweist, die gleich groß oder größer ist als der Querschnitt des Stößels (48) oder als die Auflagefläche des Tellers, wobei beim Vortreiben des Stößels (48) der Querschnitt des Stößels (48) oder die Auflagefläche des Tellers vollständig von der Anlagefläche (23) abgedeckt wird oder vorzugsweise die Anlagefläche (23) den Querschnitt des Stößels (48) oder die Auflagefläche des Tellers überragt.

## Claims

1. A storage and mixing system for pasty two-component polymethyl methacrylate bone cements, said storage and mixing system comprising a tubular cartridge (1) with a cylindrical interior,
a cartridge head (7) that closes one end of the tubular cartridge (1),
a partition wall (2) axially disposed in the cylindrical interior of the cartridge (1), wherein said partition wall (2) is connected to the circumferential surface of the cylindrical interior of the cartridge (1) and wherein the partition wall (2) divides the cylindrical interior of the cartridge (1) delimited by the cartridge head (7) into two spatially separate cavities (3, 4), wherein the first cavity (3) includes a first pasty cement component and the separate second cavity (4) includes a second pasty cement component,
two delivery plungers (5, 6) disposed movably in the axial direction in the two cavities (3, 4) of the cartridge (1), wherein said delivery plungers (5, 6) close the two cavities (3, 4) on the side of the cavities (3, 4) opposite the cartridge head (7),
wherein the delivery plungers (5, 6) on the back side opposite the cartridge head (7) are connected via a connecting means (18), wherein a wedge (22) or cone (22) having a cutting edge (20) on the front side of the wedge (22) or cone (22) facing the cartridge head (7) is disposed on the connecting means (18), such that said cutting edge (20) cuts open the partition wall (2) when the delivery plungers (5, 6) are advanced towards the cartridge head (7) in the cavities (3, 4) and the wedge (22) or cone (22) presses the cut open parts (40) of the partition wall (2) towards the inner wall of the cartridge (1).

2. The storage and mixing system according to claim 1, **characterized in that** the partition wall (2) is connected to the circumferential surface of the cylindrical interior of the cartridge (1) along two connecting lines which delimit the circumferential surface, wherein these connecting lines are preferably arranged opposite one another and the axis of the cartridge (1) particularly preferably extends in the partition wall (2).

3. The storage and mixing system according to claim 1 or 2, **characterized in that** the cavities (3, 4) have a semicircular or circular segmental cross section and the delivery plungers (5, 6) have a matching cross section, such that the delivery plungers (5, 6) close the cavities (3, 4) at each axial position in the cavities (3, 4).

4. The storage and mixing system according to any one of the preceding claims,
**characterized in that**
the delivery plungers (5, 6) are spaced apart by the connecting means (18), such that the gap between the delivery plungers (5, 6) is smaller than or equal to the thickness of the partition wall (2).

5. The storage and mixing system according to any one of the preceding claims,
**characterized in that**
the partition wall (2) has a maximum thickness of 1.5 mm, preferably a maximum thickness of 1.0 mm, and/or the partition wall (2) has such a thickness that the partition wall (2) is cuttable by the cutting edge (20) on which acts a driving force of 3 kN and is bendable by the wedge (22) or cone (22).

6. The storage and mixing system according to any one of the preceding claims,
**characterized in that**
two ducts are provided in the cartridge head (7) that connect the two cavities (3, 4) to the environment of the storage and mixing system, wherein a detachable plug (9) is disposed in each of these ducts.

7. The storage and mixing system according to any one of the preceding claims,
**characterized in that**
the cartridge (1), the cartridge head (7), the partition wall (2), and the delivery pistons (5, 6) are made of a plastic material, wherein polyethylene co-vinyl alcohol (EVOH), polybutylene terephthalate (PBT), polyethylene terephthalate (PET), and polymethacrylic acid methyl ester co-acrylonitrile are preferred plastic materials.

8. The storage and mixing system according to any one of the preceding claims,
**characterized in that**
the cartridge head (7) is realized by an rubber-elastic plate (7) which is fastened to the cartridge (1) using a sleeve cap (8), wherein said sleeve cap (8) blocks movement of the rubber-elastic plate (7) away from the cartridge (1) by means of a projecting rim, and wherein said rubber-elastic plate (7) has a recess for receiving the long side of the partition wall (2) on the side facing the delivery plungers (5, 6), and wherein said receptacle in the rubber-elastic plate (7) preferably defines two areas, wherein a duct that is closed by a plug (9) is disposed in each area.

9. The storage and mixing system according to any one of the preceding claims,
**characterized in that**
a sleeve cap (8) is provided as a connecting element for connecting the cartridge head (7) and the cartridge (1), wherein said sleeve cap (8) includes a female thread (34) or a male thread or a bayonet joint or latching elements.

10. The storage and mixing system according to any one of the preceding claims,
**characterized in that**
the connecting means (18) is designed such that the cut open parts (40) of the partition wall (2) remain connected to the inner wall of the cartridge (1) when the connecting means (18) is advanced towards the cartridge head (7) in the inner space of the cartridge (1).

11. A method for mixing pasty cement components of a pasty cement dough, particularly a polymethyl methacrylate bone cement, using a storage and mixing system according to at least one of the preceding claims, **characterized by** the following steps that take place successively:
a) removing the cartridge head (7) from the cartridge (1) or removing at least two plugs (9) from at least two ducts in the cartridge head (7), such that the cartridge (1) is opened,
b) arranging and connecting a delivery pipe (28) to the opened cartridge (1), wherein said delivery pipe (28) includes a mixer (30),
c) inserting the cartridge (1) into a manually operated delivery device, said delivery device comprising a tappet (48) that is drivable in the axial direction to advance the delivery plungers (5, 6) in the cavities (3, 4) of the cartridge (1),
d) extruding the pasty cement components by means of the delivery device through axial advancement of the delivery plungers (5, 6) using the tappet (48), by which the cement components are pressed into the delivery pipe (28), wherein the two cement components are mixed into a pasty cement dough by the mixer (30) in the delivery pipe (28), wherein the partition wall (2) is cut open in the longitudinal direction of the cartridge (1) by the cutting edge (20) in sync with the movement of the delivery plungers (5, 6), and the two cut open parts (40) of the partition wall (2) are pressed by the wedge (22) or cone (22) towards the inner wall of the cartridge (1) or at least outwards to such an extent that any further movement of the tappet (48) of the delivery device is not prevented or hindered by the parts of the cut open partition wall (2).

12. The method according to Claim 11, **characterized in that** the two cut-open parts (40) of the partition wall (2) remain connected to the inner wall of the cartridge (1).

13. The method according to Claim 11 or 12, **characterized in that** the delivery pipe (28) is connected to the cartridge (1) by connecting the connecting element (34) of the delivery pipe (28) to a connecting element (14) of the cartridge (1).

14. The method according to any one of Claims 11 to 13, **characterized in that** the tappet (48) of the delivery device also applies pressure to the side (23) of the wedge (22) or cone (22) that faces away from the delivery plungers (5, 6) and that the delivery plungers (5, 6) are driven via the wedge (22) or cone (22) and the connecting means (18).

15. The method according to any one of Claims 11 to 14, **characterized in that** the side (23) of the wedge (22) or cone (22) that faces away from the delivery plungers (5, 6) comprises a contact surface (23) for making contact with the front side of the tappet (48) or of a tray attached to the same, said contact surface being of equal size or greater than the cross section of the tappet (48) or the support surface of the tray, wherein said contact surface (23) completely covers the cross section of the tappet (48) or the support surface of the tray when the tappet (48) is advanced, or preferably said contact surface (23) exceeds the cross section of the tappet (48) or the supporting surface of the tray.

## Revendications

1. Système de stockage et de mélange pour ciments osseux à base de polyméthyl méthacrylate bi composant sous forme pâteuse, le système de stockage et de mélange présentant
une cartouche (1) en forme de tube dotée d'un espace intérieur cylindrique,
une tête de cartouche (7) qui ferme une extrémité de la cartouche (1) en forme de tube,
une paroi de séparation (2) disposée axialement dans l'espace intérieur cylindrique de la cartouche (1), la paroi de séparation (2) étant reliée avec la surface d'enveloppe de l'espace intérieur cylindrique de la cartouche (1) et la paroi de séparation (2) divisant l'espace intérieur cylindrique de la cartouche (1) délimité par la tête de cartouche (7) en deux cavités (3, 4) séparées spatialement l'une de l'autre, où un premier composant de ciment de forme pâteuse est contenu dans la première cavité (3) et un deuxième composant de ciment de forme pâteuse est contenu dans la seconde cavité (4) séparée,
deux pistons d'évacuation (5, 6)) disposés en pouvant coulisser axialement dans les deux cavités (3, 4) de la cartouche (1), où les pistons d'évacuation (5, 6) ferment les deux cavités (3, 4) sur le côté des cavités (3, 4) se situant en face de la tête de cartouche (7),
les pistons d'évacuation (5, 6) étant reliés ensemble sur le côté arrière situé en face de la tête de cartouche (7) par le biais d'un moyen de liaison (18), où un coin (22) ou un cône (22) avec un système coupant (20) est disposé sur la face avant du coin (22) ou du cône (22) orientée vers la tête de cartouche (7) de sorte que, lors d'une avancée des pistons d'évacuation (5, 6) dans les cavités (3, 4) en direction de la tête de cartouche (7), le système coupant (20) découpe la paroi de séparation (2) et le coin (22) ou le cône (22) presse les parties (40) découpées de la paroi de séparation (2) en direction de la paroi intérieure de la cartouche (1).

2. Système de stockage et de mélange selon la revendication 1, caractérisé en ce la paroi de séparation (2) est reliée le long de deux lignes de liaison qui délimitent la surface d'enveloppe, avec la surface d'enveloppe de l'espace intérieur cylindrique de la cartouche (1), où de préférence les lignes de liaison sont disposées l'une en face de l'autre et de manière particulièrement préférée, l'axe de la cartouche (1) se situe dans la paroi de séparation (2).

3. Système de stockage et de mélange selon la revendication 1 ou la revendication 2,
caractérisé en ce
les cavités (3, 4) présentent une section transversale en forme de demi cercle ou en forme de segment de cercle et les pistons d'évacuation (5, 6) présentent une section transversale y étant assortie de sorte que les pistons d'évacuation (5, 6) ferment les cavités (3, 4) dans chaque position axiale dans les cavités (3, 4).

4. Système de stockage et de mélange selon l'une des revendications précédentes,
**caractérisé en ce que**
les pistons d'évacuation (5, 6) sont espacés l'un de l'autre par le biais du dispositif de liaison (18) de telle manière que la fente située entre les pistons d'évacuation (5, 6) est plus petite ou est de la même taille que l'épaisseur de la paroi de séparation (2).

5. Système de stockage et de mélange selon l'une des revendications précédentes,
**caractérisé en ce que**
la paroi de séparation (2) a une épaisseur d'au maximum 1,5 mm, de préférence d'au maximum 1,0 mm, et/ou la paroi de séparation (2) a une épaisseur telle que la paroi de séparation (2) est à découper avec le système coupant (20) sur lequel agit une force de propulsion de 3 kN et est à plier vers le haut à partir du coin (22) ou du cône (22).

6. Système de stockage et de mélange selon l'une des revendications précédentes,
**caractérisé en ce que**
deux passages de part en part sont prévus dans la tête de cartouche (7), qui relient les deux cavités (3, 4) avec le voisinage du système de stockage et de mélange, un bouchon (9) amovible étant disposé respectivement dans les passages de part en part.

7. Système de stockage et de mélange selon l'une des revendications précédentes,
**caractérisé en ce que**
la cartouche (1), la tête de cartouche (7), la paroi de séparation (2) et les pistons d'évacuation (5, 6) sont réalisés en matière plastique, le copolymère polyéthylène alcool vinylique (EVOH), le polybutylène téréphtalate (PBT), le polyéthylène téréphtalate (PET) et le copolymère de polyméthacrylate de méthyle acrylonitrile étant préférés en tant que matières plastiques.

8. Système de stockage et de mélange selon l'une des revendications précédentes,
**caractérisé en ce que**
la tête de cartouche (7) est réalisée par une plaque (7) en caoutchouc élastique qui est fixée sur la cartouche (1) avec un bouchon à vis (8), où le bouchon à vis (8) bloque un mouvement d'éloignement à partir de la cartouche (1) de la plaque (7) en caoutchouc élastique à l'aide d'un rebord protubérant, et où la plaque (7) en caoutchouc élastique possède un renfoncement sur le côté orienté vers les piston d'évacuation (5, 6) pour la réception du côté longitudinal de la paroi de séparation (2), et où de préférence deux zones sont définies par ce logement dans la plaque (7) en caoutchouc élastique, où un passage de part en part, qui est fermé par un bouchon (9), est disposé dans chaque zone.

9. Système de stockage et de mélange selon l'une des revendications précédentes,
**caractérisé en ce que**
le bouchon à vis (8) est prévu en tant qu'élément de liaison pour la liaison de la tête de cartouche (7) avec la cartouche (1), le bouchon à vis (8) présentant un filetage intérieur (34) ou un filetage extérieur ou une fermeture à baïonnette ou des éléments de verrouillage.

10. Système de stockage et de mélange selon l'une des revendications précédentes,
**caractérisé en ce que**
le moyen de liaison (18) est conçu de manière à ce que les deux parties (40) découpées de la paroi de séparation (2) restent reliées avec la paroi intérieure de la cartouche (1) lorsque le moyen de liaison (18) est avancé dans l'espace intérieur de la cartouche (1) en direction de la tête de cartouche (7).

11. Procédé de mélange de composants de ciment sous forme pâteuse d'une pâte de ciment en forme pâteuse, notamment d'un ciment osseux à base de polyméthyl méthacrylate, avec un système de stockage et de mélange selon au moins une des revendications précédentes, **caractérisé par** les étapes suivantes se déroulant l'une après l'autre,
a) le retrait de la tête de cartouche (7) de la cartouche (1) ou le retrait d'au moins deux bouchons (9) à partir d'au moins deux passages de part en part dans la tête de cartouche (7), de sorte que la cartouche (1) est ouverte,
b) le montage et la mise en communication d'un tube d'évacuation (28) avec la cartouche (1) ouverte, où le tube d'évacuation (28) contient un mélangeur (30),
c) l'insertion de la cartouche (1) dans un dispositif d'évacuation à actionner manuellement, le dispositif d'évacuation présentant un poussoir (48) à actionner axialement pour pousser les pistons d'évacuation (5, 6) vers l'avant dans les cavités (3, 4) de la cartouche (1),
d) le pressage des composants de ciment de forme pâteuse à l'aide du dispositif d'évacuation par un avancement axial des pistons d'évacuation (5, 6) avec le poussoir (48), ce par quoi les composants de ciment sont pressés dans le tube d'évacuation (28), où les deux composants de ciment sont mélangés par le mélangeur (30) dans le tube d'évacuation (28) en une pâte de ciment de forme pâteuse, où, de manière synchronisée au déplacement des pistons d'évacuation (5, 6), la paroi de séparation (2) est découpée avec le système coupant (20) dans le sens longitudinal de la cartouche (1) et les deux parties (40) découpées de la paroi de séparation (2) sont pressées avec le coin (22) ou le cône (22) en direction de la paroi intérieure de la cartouche (1) au moins aussi loin vers l'extérieur qu'un nouveau déplacement du poussoir (48) du dispositif d'évacuation n'est pas empêché ou n'est pas gêné par les parties (40) de la paroi de séparation (2) découpée.

12. Procédé selon la revendication 11, **caractérisé en ce que**
les deux parties (40) découpées de la paroi de séparation (2) restent reliées avec la paroi intérieure de la cartouche (1).

13. Procédé selon l'une des revendications 11 ou 12, **caractérisé en ce que**
le tube d'évacuation (28) est relié avec la cartouche (1) par la connexion de l'élément de liaison (34) du tube d'évacuation (28) avec un élément de liaison (14) de la cartouche (1).

14. Procédé selon l'une des revendications 11 à 13, **caractérisé en ce que**
le poussoir (48) du dispositif d'évacuation presse sur le côté (23) du coin (22) ou du cône (22) opposé aux pistons d'évacuation (5, 6) et les pistons d'évacuation (5, 6) sont entraînés par le biais du coin (22), ou du cône (22) et du moyen de liaison (18).

15. Procédé selon l'une des revendications 11 à 14, **caractérisé en ce que**
le côté (23) du coin (22) ou du cône (22) opposé aux pistons d'évacuations (5, 6) présente une surface d'appui (23) pour la mise en appui du côté avant du poussoir (48) ou d'une partie plane y étant rapportée qui est de la même taille ou plus grande que la section transversale du poussoir (48) ou que la surface d'appui de la partie plane, où, lors de l'avancée du poussoir (48), la section transversale du poussoir (48), ou la surface d'appui de la partie plane, est totalement recouverte par la surface d'appui (23), ou, de préférence, la surface d'appui (23) dépasse au-dessus de la section transversale du poussoir (48) ou la surface d'appui de la partie plane.
